(19)

Europäisches
Patentamt
European
Patent Office
Office européen
des brevets

(11) **EP 4 442 352 A1**

(12) **EUROPEAN PATENT APPLICATION**
published in accordance with Art. 153(4) EPC

(43) Date of publication:
**09.10.2024 Bulletin 2024/41**

(21) Application number: **21966303.6**

(22) Date of filing: **30.11.2021**

(51) International Patent Classification (IPC):
**B01D 61/02** (2006.01)     **B01D 61/26** (2006.01)
**B01D 61/32** (2006.01)     **B01D 61/58** (2006.01)
**A61M 1/16** (2006.01)

(52) Cooperative Patent Classification (CPC):
**A61M 1/16; A61M 1/1656; B01D 61/02;
B01D 61/32; B01D 61/58**

(86) International application number:
**PCT/JP2021/043741**

(87) International publication number:
**WO 2023/100216 (08.06.2023 Gazette 2023/23)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Toray Industries, Inc.
Tokyo 103-8666 (JP)**

(72) Inventors:
• **YOSHIDOMI, Shinichiro
  Otsu-shi, Shiga 520-8558 (JP)**

• **NAGANO, Izumi
  Otsu-shi, Shiga 520-8558 (JP)**
• **MIYAMOTO, Ryoma
  Otsu-shi, Shiga 520-8558 (JP)**
• **OKAMOTO, Yoshiki
  Otsu-shi, Shiga 520-8558 (JP)**
• **SHIMURA, Harutoki
  Otsu-shi, Shiga 520-8558 (JP)**
• **HANADA, Shigehisa
  Otsu-shi, Shiga 520-8558 (JP)**

(74) Representative: **Kador & Partner Part mbB
Corneliusstraße 15
80469 München (DE)**

(54) **SOLUTION PROCESSING METHOD AND SOLUTION PROCESSING APPARATUS**

(57)     An object of the present invention is to provide a solution treatment method which is to be applied to a solution containing at least an electrolyte in an amount of 1000 mg/L or more and containing a low-molecular-weight nonelectrolyte molecule having a molecular weight of 70 or less, and by which the electrolyte and water are recovered while the low-molecular-weight nonelectrolyte molecule is removed, with combination of a plurality of types of membranes. The present invention provides a solution treatment method comprising treating a raw liquid with a separation membrane 1 and a separation membrane 2 which each separate a feed liquid into a permeate liquid and a concentrated liquid, and separating the raw liquid into a purified liquid containing water and an electrolyte and a discharge liquid containing a low-molecular-weight nonelectrolyte molecule, wherein the raw liquid is a solution containing at least an electrolyte in an amount of 1000 mg/L or more and containing a low-molecular-weight nonelectrolyte molecule having a molecular weight of 70 or less, a permeate liquid from the separation membrane 1 is fed to the separation membrane 2 and subjected to separation treatment,

the separation membrane 1 exhibits, when a solution 1 with pH 7 in which 10000 mg/L of sodium chloride (NaCl) and 250 mg/L of urea are mixed is fed thereto at 36°C and a pressure of 1.2 Mpa, an NaCl removal rate of 90% or more, the NaCl removal rate being defined by a reduction rate of a component concentration in a permeated solution relative to a component concentration in a feed liquid,

the separation membrane 2 exhibits, when a solution 2 with pH 7 in which 1000 mg/L of sodium chloride (NaCl) and 700 mg/L of urea are mixed is fed thereto at 36°C and a pressure of 1.8 Mpa, a urea removal rate of 85% or more, the urea removal rate being defined by a reduction rate of a component concentration in a permeated solution relative to on a component concentration in a feed liquid,

a difference between a urea removal rate of the separation membrane 2 under the evaluation conditions of the separation membrane 2 specified above and a urea removal rate of the separation membrane 1 under the evaluation conditions of the separation membrane 1 specified above is 40% point or more, and

the solution treatment method satisfies any one of the following requirements (i) and (ii):

(i) a part of a permeate liquid from the separation membrane 2 is mixed with the raw liquid and then fed to the separation membrane 1, and a whole or a part of the remainder of the permeate liquid from the separation membrane 2 is mixed with a concentrated liquid from the separation membrane 1 to afford the purified liquid; and

(ii) a whole of a permeate liquid from the separation membrane 2 is mixed with the raw liquid and then fed to the separation membrane 1 to afford a concentrated liquid from the separation membrane 1 as the purified liquid.

【Fig.1】

**Description**

TECHNICAL FIELD

The present invention relates to a solution treatment method utilizing membrane separation by which method, from a solution containing at least an electrolyte in an amount of 1000 mg/L or more and containing a low-molecular-weight nonelectrolyte molecule having a molecular weight of 70 or less, the electrolyte and water are recovered while the low-molecular-weight nonelectrolyte molecule is separated.

BACKGROUND ART

In recent years, there is an increasing need for selective separation techniques for extracting a useful substance from a solution in which a plurality of components are dissolved or for separating impurities. For example, valuable substances are taken out from salt lake water in which various elements are dissolved, impurities are taken out from a solution containing impurities, and the like are demanded. More specifically, for example, Patent Document 1 describes a method of removing boron dissolved in fresh water obtained by sea water desalination, raw water used for tap water, or the like using a reverse osmosis membrane and a loose RO membrane. In addition, Patent Document 2 describes a method of removing salts while leaving mineral components from sea water or deep ocean water using a reverse osmosis membrane and a nanofiltration (NF) membrane.

Under needs for various selective separation techniques, in recent years, the number of patients on artificial dialysis treatment is increasing worldwide along with the improvement of living standards, and a technique for removing impurities from a waste dialysate discharged via treatment and regenerating the waste dialysate as a dialysate is attracting attention. Common artificial dialysis treatment requires the use of a large quantity of dialysate as large as 90 L to 150 L in a single treatment. Since a dialysate used for artificial dialysis treatment contains waste products such as urea transferred from the blood, the dialysate is basically discarded via a single use. Therefore, there is a problem that a large amount of waste dialysate is generated.

As a method of preparing a dialysate, a method of adding a necessary electrolyte, glucose, or the like to pure water obtained by treating tap water with a reverse osmosis (RO) membrane or the like to produce a dialysate is common. However, in a water shortage area where tap water is supplied intermittently, it may be difficult to secure a necessary amount of tap water, and a reduction in water usage is required. Furthermore, even in an area where water is supplied sufficiently, if water supply is stopped continuously at the time of disaster, dialysate cannot be made from tap water. Therefore, there is an increasing demand for reuse of a waste dialysate by removing waste products such as urea from the waste dialysate and reusing the treated waste dialysate as a dialysate, and several proposals of waste dialysate reuse technique have been made.

For example, it has been proposed to remove impurities, waste products, and electrolytes from a used waste dialysate with an adsorbent. However, depending on the dialysis treatment to be performed, several kilograms of adsorbent is needed for regeneration of a waste dialysate, and a system that suppresses weight and cost as much as possible is desired.

As a method for reducing the amount of an adsorbent, Patent Document 3 proposes a system using an adsorbent cartridge in two stages.

Patent Document 4 proposes a urea removal system using urease and an ion exchange resin or an inorganic adsorbent.

Patent Document 5 proposes a system that removes impurities using a reverse osmosis membrane after removing electrolytes to some extent with an adsorbent or by electrodialysis.

PRIOR ART DOCUMENTS

PATENT DOCUMENTS

Patent Document 1: JP H09-290275 A
Patent Document 2: JP 2003-088863 A
Patent Document 3: JP 2014-204958 A
Patent Document 4: JP 2014-530643 A
Patent Document 5: WO 2020/218571 A

## SUMMARY OF THE INVENTION

## PROBLEMS TO BE SOLVED BY THE INVENTION

The techniques described in Patent Documents 3 and 4 are techniques for separating urea from a used dialysate by decomposing urea into ammonia with urease and further capturing ammonia. Therefore, a plurality of adsorbents is required to completely capture urea and ammonia which is a decomposition product of the urea. Because of a concern that ammonia that has not been captured may remain if the amount of the adsorbent is small, a large amount of the adsorbent is required, which has caused a problem that the cost increases or the weight of a waste dialysate recycling device increases.

The technique described in Patent Document 5 is a technique for separating waste products such as urea using a reverse osmosis membrane having a pore diameter of 7.0 Å or less and regenerating a waste dialysate. In this technique, particularly when water is treated with high recovery, it is necessary to remove electrolytes in a waste dialysate as pretreatment in order to reduce an osmotic pressure difference in a reverse osmosis membrane, and an ion exchange resin or an electrodialyzer is used as the pretreatment. However, when an ion exchange resin is used, there is a problem that the waste of the ion exchange resin used in a single dialysis treatment is discharged. In addition, when an electrodialyzer is used, this is unfavorable in terms of safety because a high voltage is required for desalination and there is a concern of chlorine gas generation.

In view of the above, an object of the present invention is to provide a solution treatment method in which a solution containing a separation target substance such as a neutral molecule is treated with a combination of a plurality of types of membranes, and a component and water desired to be left as a purified liquid are recovered while the unnecessary separation target substance is separated. More specifically, it is an object of the present invention to provide a solution treatment method which is to be applied to a solution containing at least an electrolyte in an amount of 1000 mg/L or more and containing a low-molecular-weight nonelectrolyte molecule having a molecular weight of 70 or less, and by which the electrolyte and water are recovered while the low-molecular-weight nonelectrolyte molecule is removed, with combination of a plurality of membranes.

## SOLUTIONS TO THE PROBLEMS

To attain the above object, the present invention mainly employs any one of the following configurations.

(1) A solution treatment method comprising treating a raw liquid with a separation membrane 1 and a separation membrane 2 which each separate a feed liquid into a permeate liquid and a concentrated liquid, and separating the raw liquid into a purified liquid containing water and an electrolyte and a discharge liquid containing a low-molecular-weight nonelectrolyte molecule, wherein

the raw liquid is a solution containing at least an electrolyte in an amount of 1000 mg/L or more and containing a low-molecular-weight nonelectrolyte molecule having a molecular weight of 70 or less,
a permeate liquid from the separation membrane 1 is fed to the separation membrane 2 and subjected to separation treatment,
the separation membrane 1 exhibits, when a solution 1 with pH 7 in which 10000 mg/L of sodium chloride (NaCl) and 250 mg/L of urea are mixed is fed thereto at 36°C and a pressure of 1.2 Mpa, an NaCl removal rate of 90% or more, the NaCl removal rate being defined by a reduction rate of a component concentration in a permeated solution relative to a component concentration in a feed liquid,
the separation membrane 2 exhibits, when a solution 2 with pH 7 in which 1000 mg/L of sodium chloride (NaCl) and 700 mg/L of urea are mixed is fed thereto at 36°C and a pressure of 1.8 Mpa, a urea removal rate of 85% or more, the urea removal rate being defined by a reduction rate of a component concentration in a permeated solution relative to a component concentration in a feed liquid,
a difference between a urea removal rate of the separation membrane 2 under the evaluation conditions of the separation membrane 2 specified above and a urea removal rate of the separation membrane 1 under the evaluation conditions of the separation membrane 1 specified above is 40% point or more, and
the solution treatment method satisfies any one of the following requirements (i) and (ii):

(i) a part of a permeate liquid from the separation membrane 2 is mixed with the raw liquid and then fed to the separation membrane 1, and a whole or a part of the remainder of the permeate liquid from the separation membrane 2 is mixed with a concentrated liquid from the separation membrane 1 to afford the purified liquid; and
(ii) a whole of a permeate liquid from the separation membrane 2 is mixed with the raw liquid and then fed to the separation membrane 1 to afford a concentrated liquid from the separation membrane 1 as the purified liquid.

(2) A solution treatment unit comprising a separation membrane 1 and a separation membrane 2 each of which separates a feed liquid into a permeate liquid and a concentrated liquid, and being configured to separate a raw liquid into a purified liquid containing water and an electrolyte and a discharge liquid containing a low-molecular-weight nonelectrolyte molecule, wherein

the raw liquid is a solution containing at least an electrolyte in an amount of 1000 mg/L or more and containing a low-molecular-weight nonelectrolyte molecule having a molecular weight of 70 or less,
a permeate liquid line from the separation membrane 1 is connected to a feed liquid line to the separation membrane 2,
the separation membrane 1 exhibit, when a solution 1 with pH 7 in which 10000 mg/L of sodium chloride (NaCl) and 250 mg/L of urea are mixed is fed thereto at 36°C and a pressure of 1.2 Mpa, an NaCl removal rate of 90% or more, the NaCl removal rate being defined by a reduction rate of a component concentration in a permeated solution relative to a component concentration in a feed liquid,
the separation membrane 2 exhibits, when a solution 2 with pH 7 in which 1000 mg/L of sodium chloride (NaCl) and 700 mg/L of urea are mixed is fed thereto at 36°C and a pressure of 1.8 Mpa, a urea removal rate of 85% or more, the urea removal rate being defined by a reduction rate of a component concentration in a permeated solution relative to a component concentration in a feed liquid,
a difference between a urea removal rate of the separation membrane 2 under the evaluation conditions of the separation membrane 2 specified above and a urea removal rate of the separation membrane 1 under the evaluation conditions of the separation membrane 1 specified above is 40% point or more, and
the solution treatment method satisfies any one of the following requirements (i) and (ii):

(i) a permeate liquid line from the separation membrane 2 is branched into at least two lines, at least one of the branched lines is connected to a line of the raw liquid, a line of a mixed liquid of the raw liquid and a permeate liquid from the separation membrane 2 is connected to a feed line to the separation membrane 1, and at least one of the remaining branched lines is connected to a concentrated liquid line from the separation membrane 1 to form a purified liquid line; and
(ii) a permeate liquid line from the separation membrane 2 is connected to a line of the raw liquid, a line of a mixed liquid of the raw liquid and a permeate liquid from the separation membrane 2 is connected to a feed line to the separation membrane 1, and a concentrated liquid line from the separation membrane 1 serves as a purified liquid line.

It is noted that the "raw liquid" refers to a solution fed to the most upstream of the present invention, and a solution fed to each separation membrane is referred to as a "feed liquid" based on the separation membrane.

EFFECTS OF THE INVENTION

In accordance with the solution treatment method of the present invention, by performing a specific membrane separation treatment on a solution containing a separation target substance, it is possible to recover a component desired to be recovered while separating the separation target substance. In addition, for example, when the membrane separation treatment is performed on a waste dialysate after being subjected to artificial dialysis treatment, an electrolyte such as a salt and water can be recovered while urea is separated. Therefore, it is possible to reuse the waste dialysate, and it becomes possible to stabilize the supply of dialysate or control the dialysis medical cost.

BRIEF DESCRIPTION OF THE DRAWINGS

Fig. 1 is a schematic diagram of a solution treatment method illustrating one example of the present invention.
Fig. 2 is a schematic diagram of a solution treatment method illustrating another example of the present invention.
Fig. 3 is a partially developed perspective view of an I-type separation membrane element that can be used in the present invention.
Fig. 4 is a schematic view illustrating one example of an I-type separation membrane body.
Fig. 5 is a schematic view illustrating one example of a reverse L-type separation membrane body.
Fig. 6 is a schematic view illustrating one example of an L-type separation membrane body.

Fig. 7 is a schematic view (sectional view) illustrating one example of a reverse L-type separation membrane element.

Fig. 8 is a schematic view (sectional view) illustrating one example of an L-type separation membrane element.

Fig. 9 is a schematic view (sectional view) illustrating one example of a U-turn type (I-type-reverse L-type) separation membrane element.

Fig. 10 is a schematic view (sectional view) illustrating one example of the structure of a separation membrane module including an I-type separation membrane element.

Fig. 11 is a schematic view (sectional view) illustrating one example of a separation membrane module including an I-type separation membrane element and a reverse L-type separation membrane element.

Fig. 12 is a schematic view (sectional view) illustrating one example of a separation membrane module including an I-type separation membrane element and an L-type separation membrane element.

Fig. 13 is a schematic view (sectional view) illustrating one example of a separation membrane module including an I-type separation membrane element and a U-turn type separation membrane element.

Fig. 14 is a schematic diagram of a dialysis system including a solution treatment unit of the present invention and a dialyzer.

EMBODIMENTS OF THE INVENTION

Hereinafter, embodiments of the present invention will be described in detail with reference to the drawings, but the present invention is not limited thereto at all.

(1) Raw liquid

In the present invention, the raw liquid is a solution containing at least an electrolyte in an amount of 1000 mg/L or more and containing a low-molecular-weight nonelectrolyte molecule having a molecular weight of 70 or less (hereinafter sometimes simply referred to as "low-molecular-weight nonelectrolyte molecule"), and examples of such a solution include a waste dialysate containing urea as a low-molecular-weight nonelectrolyte molecule, and sea water or brackish water containing boron as a low-molecular-weight nonelectrolyte molecule.

Examples of the electrolyte include sodium ion, potassium ion, calcium ion, magnesium ion, chloride ion, sulfate ion, and nitrate ion, and examples of the low-molecular-weight nonelectrolyte molecule having a molecular weight of 70 or less include urea, boron, methanol, and ethanol. These can be confirmed by analyzing components by such a method as ion chromatography or liquid chromatography. As described later, in order for the separation membrane 1 to exhibit sufficient selective separation performance, the molecular weight of the low-molecular-weight nonelectrolyte molecule contained in the raw liquid needs to be 70 or less. The concentration of the low-molecular-weight nonelectrolyte molecule in the raw liquid is preferably 10 mg/L or more, more preferably 100 mg/L or more, and still more preferably 500 mg/L or more in order to sufficiently exhibit the selective separation performance of the present invention.

In the following, dialysis and waste dialysate will now be described in detail.

Publicly-known methods are applied for dialysis. A dialyzer has a dialysis membrane that permeates toxins such as urea and does not permeate plasma components. While a dialysate is fed to one side of the dialysis membrane, blood is fed to the other side. The composition of the dialysate is known, and includes sodium ion, potassium ion, calcium ion, magnesium ion, glucose, and so on. Urea in the blood is diffused into the dialysate through the dialysis membrane, whereby urea is removed from the blood. Besides urea, potassium ion is one of the substances that need to be removed by dialysis. Since about 90% of potassium ion are excreted from the kidneys, dialysis patients are likely to accumulate potassium ion in the body. Hyperpotassemia can cause cardiac arrest, and controlling blood potassium ion concentration is also an important role in dialysis. As a dialysis system, it is necessary to remove blood potassium ion in a range of 26% to 35% in every dialysis and to control the blood potassium ion concentration.

As the waste dialysate that has passed through the dialyzer, for example, one containing 10000 mg/L of electrolyte including 100 mg/L of potassium ion, and about 600 mg/L of urea, which is a low-molecular-weight nonelectrolyte molecule, is assumed. In the present invention, it is preferable to treat such a waste dialysate as a raw liquid. Then, a solution containing a small amount of an electrolyte or the like is added to the purified liquid obtained by the present invention to yield a regenerated dialysate. The regenerated dialysate preferably has a urea concentration reduced by 65% or more and potassium ion reduced by 2% or more with respect to the waste dialysate, and in order to reduce the amount of the electrolyte to be added, the purified liquid preferably has recovered 80% or more of the electrolyte in the waste dialysate.

(2) Separation membrane

<Overview>

In the present invention, the raw liquid is treated using at least two types of separation membranes. Each of the two types of separation membranes may be a single layer, or may be a composite membrane including a separation functional layer and a substrate. In addition, in the composite membrane, there may be a porous support layer between the separation functional layer and the substrate.

The two types of membranes, that is, the separation membrane 1 and the separation membrane 2 are disposed such that the permeate liquid from the separation membrane 1 is fed to the separation membrane 2. In order to ensure a required electrolyte removal rate (described later), the separation membrane 1 preferably has a pore diameter of 7 Å or more as measured using a positron annihilation lifetime spectroscopy. On the other hand, in order to ensure a required electrolyte removal rate (described later), the separation membrane 2 preferably has a pore diameter of 7 Å or less as measured using a positron annihilation lifetime spectroscopy. When the pore diameter of the separation membrane 2 is 7 Å or less, the separation membrane 2 can highly remove such components as urea and electrolytes.

The pore diameter is measured using a positron annihilation lifetime spectroscopy. The positron annihilation lifetime spectroscopy is a method of measuring a time period (on an order of several hundreds of picoseconds to several tens of nanoseconds) from the incidence of a positron on a sample to the annihilation of the positron, and nondestructively evaluating information such as a size of a pore of 0.1 to 10 nm, a number density thereof, and distribution of the size thereof, on the basis of the annihilation lifetime. This measurement method is described in "Experimental Chemistry Lecture 4th Edition", volume 14, page 485, edited by the Chemical Society of Japan, Maruzen Co., Ltd. (1992).

When the separation membrane is a composite membrane, the average pore radius R of the separation functional layer is determined from the following formula (1) on the basis of a positron annihilation lifetime $\tau$ described above. Formula (1) shows a relationship in a case where it is assumed that o-Ps (ortho-positronium) is present in a pore having a radius R in an electron layer having a thickness $\Delta R$, and $\Delta R$ is empirically determined to be 0.166 nm (details thereof are described in Nakanishi, etc., Journal of Polymer Science, Part B: Polymer Physics, Vol. 27, p. 1419, John Wiley & Sons, Inc. (1989)).

[Formula 1]

$$\tau^{-1} = 2\left[1 - \frac{R}{R + \Delta R} + \frac{1}{2\pi}\sin\left(\frac{2\pi R}{R + \Delta R}\right)\right] \qquad (1)$$

In the present invention, it is necessary that the separation membranes 1 and 2 have the salt removal rate and the urea removal rate specified below, respectively.

First, the salt removal rate and the urea removal rate of the separation membrane 1 are each defined by the reduction rate of the concentration of the target component in the permeated solution relative to the concentration of the target component in the fed solution when a solution 1 with pH 7 in which 10000 mg/L of sodium chloride (NaCl) and 250 mg/L of urea are mixed is fed at a temperature of 36°C and a pressure of 1.2 MPa.

The salt removal rate and the urea removal rate of the separation membrane 2 are each defined by the reduction rate of the concentration of the target component in the permeated solution relative to the concentration of the target component in the fed solution when a solution 2 with pH 7 in which 1000 mg/L of sodium chloride (NaCl) and 700 mg/L of urea are mixed is fed at a temperature of 36°C and a pressure of 1.8 MPa.

Under the evaluation conditions described above, a flow rate on membrane surface with which the recovery rate, which is the ratio of the amount of the permeate liquid to the amount of the feed liquid to be fed, can be approximated to 0, namely, a sufficient flow rate on membrane surface is required.

The concentration of urea is measured using the urease GLDH method. The urease GLDH method is a method for measuring urea nitrogen (BUN) by performing the following first reaction and second reaction and measuring the change in the amount of a coenzyme (NADPH).

(First reaction)

In the following reaction formula (II), the endogenous ammonia generated via the following reaction formula (I) is eliminated by the action of α-ketoglutaric acid, reduced nicotinamide adenine dinucleotide phosphate (NADPH), and glutamic acid dehydrogenase (GLDH), and the oxidized nicotinamide adenine dinucleotide phosphate (NADP) generated at this time is reduced by the action of L-isocitrate dehydrogenase (ICDH) via the reaction formula (III) to change to NADPH.

(Second reaction)

After eliminating the endogenous ammonia via the first reaction, urea is decomposed into ammonia and carbon dioxide by the action of urease. The ammonia and the $\alpha$-ketoglutaric acid ($\alpha$-KG) are converted to glutamic acid by the action of GLDH, while NADPH is converted to NADP. NADPH has an absorption maximum at 340 nm, and the rate of decrease in absorbance is measured to determine a urea nitrogen value. Incidentally, at this time, the reaction formula (III) of the first reaction is stopped by the action of a chelating agent added to a second reagent.

$$\text{Urea} + H_2O \ (+ \text{urease}) \rightarrow 2NH_3 + CO_2 \ ... \qquad (I)$$

$$\alpha\text{-Ketoglutaric acid} + NH_3 + NADPH + H+ \ (+ \text{GLDH}) \rightarrow \text{glutamic acid} + NADP+ + H_2O \ ... \qquad (II)$$

$$NADP+ + \text{L-isocitric acid} \ (+ \text{ICDH}) \rightarrow NADPH+ + \alpha\text{-ketoglutaric acid} + CO_2 \ ... \qquad (III)$$

In the present invention, the sodium chloride removal rate of the separation membrane 1 measured under the evaluation conditions of the separation membrane 1 is 90% or more. In addition, in order to preferentially remove 65% or more of urea as a whole system, the urea removal rate of the separation membrane 2 measured under the evaluation conditions of the separation membrane 2 is 85% or more, and the difference between the urea removal rate of the separation membrane 2 measured under the evaluation conditions of the separation membrane 2 and the urea removal rate of the separation membrane 1 measured under the evaluation conditions of the separation membrane 1 is 40% point or more (preferably 60% or more). By satisfying these removal rates, sufficient performance can be exhibited as a whole process.

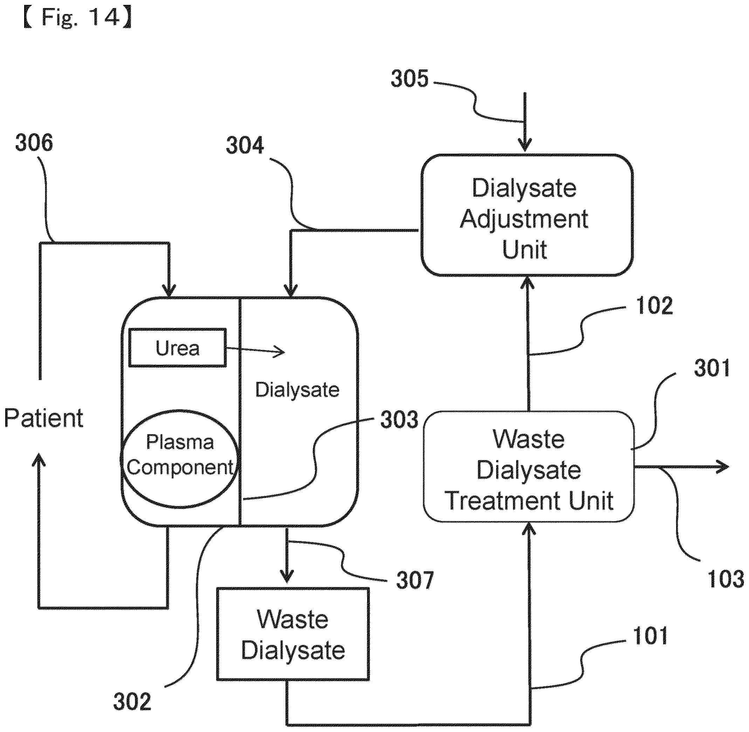

When the process of the present invention is combined with a dialyzer as shown in Fig. 14, potassium ion will be recovered together with other electrolytes and accumulated in a regenerated dialysate. However, when the present inventors have investigated the relationship between the salt removal rate of the separation membrane 1 and the blood potassium ion removal rate after dialysis for 4 hours, if the salt removal rate of the separation membrane 1 is in the range of 99% or less, potassium ion is removed by 2% or more as a whole system shown in Fig. 14, and the blood potassium ion removal rate can be controlled to 26% or more. When potassium ion is excessively removed by using a membrane having a small salt removal rate as the separation membrane 1, it is advised to add potassium ion as well to an electrolyte-containing adjustment solution to be added when a purified liquid is used as a regenerated dialysate.

In the present invention, the type of the separation membrane 1 is not particularly limited as long as the conditions described above are satisfied, but in general, a nanofiltration membrane or a low-pressure RO membrane is suitably used.

The type of the separation membrane 2 is not particularly limited as long as the conditions described above are satisfied, but a dense RO membrane to be used for sea water desalination or the like is suitably used.

<Separation functional layer>

The separation functional layer may be a layer having both a separation function and a support function, or may have only a separation function. The "separation functional layer" refers to a layer having at least a separation function.

When the separation functional layer has both a separation function and a support function, a layer containing a polymer selected from the group consisting of cellulose, polyvinylidene fluoride, polyethersulfone, and polysulfone as a main component is suitably applied as the separation functional layer.

On the other hand, only to exhibit a separation function, a crosslinked polymer is preferably used because this is easy to control the pore diameter and is superior in durability. In particular, a polyamide separation functional layer obtained via polycondensation of a polyfunctional amine and a polyfunctional acid halide, an organic-inorganic hybrid functional layer, etc. are suitably used from the viewpoint that they are superior in performance to separate components in a feed liquid. These separation functional layers can be formed via polycondensation of monomers on a porous support layer separately prepared. For example, the polyamide separation functional layer is obtained by applying an aqueous polyfunctional amine solution to a porous support layer, removing an excess aqueous polyfunctional amine solution with an air knife or the like, and then applying an organic solvent solution containing a polyfunctional acid halide to cause interfacial polycondensation.

<Porous support layer>

The porous support layer is a layer that supports the separation functional layer, and can also be referred to as a porous resin layer when the material of the layer is a resin.

The material to be used for the porous support layer and the form of the layer are not particularly limited, but, for example, the layer may be formed of a porous resin on a substrate. The composition of the porous support layer is not particularly limited, but is preferably formed of a thermoplastic resin. Here, the thermoplastic resin refers to a resin that is made of a chain polymer substance and exhibits a property of being deformed or flowed by an external force when heated. Examples of the thermoplastic resin include homopolymers or copolymers such as polysulfone, polyether sulfone, polyamide, polyester, cellulose-based polymer, vinyl polymer, polyphenylene sulfide, polyphenylene sulfide sulfone, polyphenylene sulfone, and polyphenylene oxide, and these may be used either singly or as a blend. As the cellulose-based polymer, cellulose acetate, cellulose nitrate, etc. can be used, and as the vinyl polymer, polyethylene, polypropylene, polyvinyl chloride, polyacrylonitrile, an acrylonitrile-styrene copolymer, etc. can be used. Among them, it is preferable to use polysulfone, which has high chemical, mechanical, and thermal stability and whose pore diameter is easily controlled.

The porous support layer can be produced, for example, by casting a solution of the polysulfone in N,N-dimethylformamide in a certain thickness onto a substrate (for example, a densely woven polyester nonwoven fabric) to be described later, and then wet-solidifying the solution in water.

<Substrate>

In terms of the strength, dimensional stability, and so on of a separation membrane, the separation membrane may have a substrate. As the substrate, a fibrous substrate is preferably used in terms of strength and fluid permeability. In particular, a continuous fiber nonwoven fabric and a short fiber nonwoven fabric are preferable.

(3) Separation membrane module

<Overview>

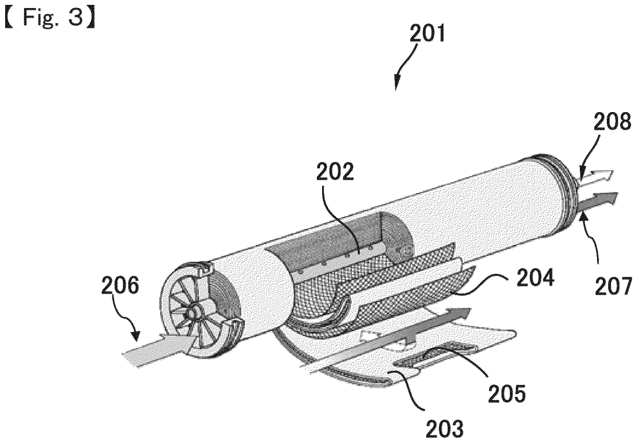

The separation membrane 1 and the separation membrane 2 in the present invention can be configured in the form of a spiral-type element in which a flat membrane-like membrane is wound around a perforated central tube, a plate-and-frame-type element in which flat membranes stretched on both sides of a plate-type support plate are laminated at regular intervals with a spacer interposed therebetween, a tubular-type element using a tubular membrane, or a hollow fiber membrane element in which hollow fiber membranes are bundled and housed in a case. Further, one or more of these elements are connected in series in a pressure-resistant container and stored to form a separation membrane module. The form of the element may be any form, but it is preferable to use a spiral-type separation membrane element as illustrated in Fig. 3 from the viewpoint of operability and compatibility. The spiral-type separation membrane element is an element in which a laminate of a separation membrane, a permeate-side channel material, and a feed-side channel material is wound around a perforated central tube for collecting a permeate liquid.

<Separation membrane body of spiral-type separation membrane element>

The flat membrane-like separation membrane has a permeate-side face and a feed-side face. A combination of a separation membrane leaf arranged with its permeate-side face facing itself with a permeate-side channel material interposed and a feed-side channel material disposed on one side of the feed-side face of the separation membrane leaf is call a separation membrane body. A permeate-side channel is formed such that a permeated fluid flows into a perforated central tube, and a space between the permeate-side faces of the separation membrane is opened at only one side lying inside in a winding direction and sealed at other three sides. The feed-side channel is formed between the feed-side faces of the separation membrane, and separation membrane elements of various types, such as I-type, L-type, reverse L-type, and U-turn-type, are formed as described later by a method of sealing a feed-side channel material.

<I-Type separation membrane element>

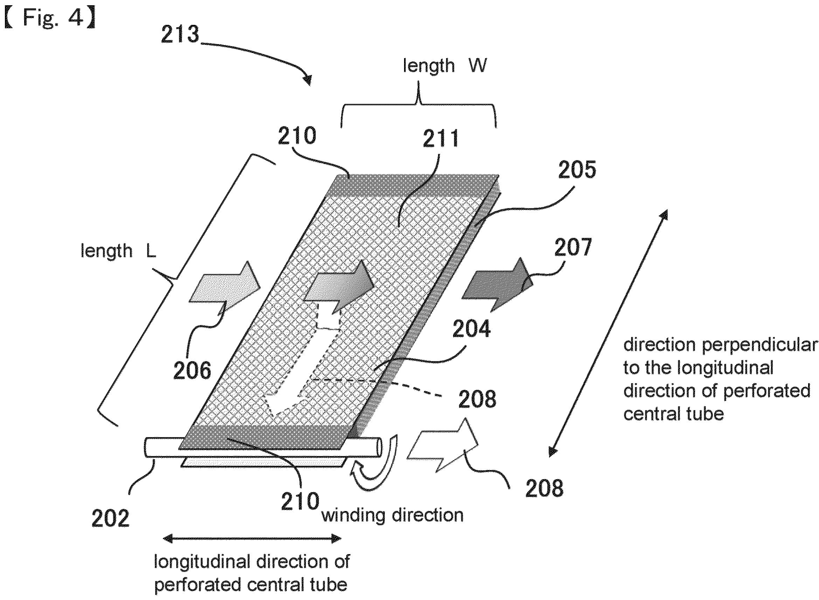

In a separation membrane element (201) called I-type, as illustrated in Fig. 3, a feed liquid (206) flows in from one end face, and a concentrated liquid (207) is discharged from the other end face. The I-type separation membrane element (201) includes a separation membrane (203), a feed-side channel material (204), a permeate-side channel material(205), and a perforated central tube (202). In the I-type separation membrane element (201), the separation membrane (203) forms a separation membrane body (213) as illustrated in Fig. 4 together with the feed-side channel material (204) and the permeate-side channel material (205). The separation membrane (203) is arranged such that the permeate-side face faces itself with the permeate-side channel material (205) interposed. In addition, on a feed-side face (211) of the separation membrane (namely, between the feed-side face and a feed-side face of a separation membrane constituting an adjacent other separation membrane body), the feed-side channel material (204) is disposed. The permeate-side channel is formed with a space between the permeate-side faces of the separation membranes constituting two adjacent separation membrane bodies being opened only at one side in the winding direction and sealed (closed) at the other three sides such that a permeate liquid (208) flows into the perforated central tube (202). The feed-side channel material is disposed such that it is sandwiched by the feed-side face of the separation membrane, and forms a channel for feeding a feed liquid to the separation membrane (namely, a feed-side channel).

In addition, the feed-side channel material preferably has a form that makes the flow of the feed liquid turbulent in order to inhibit concentration polarization of the feed liquid. Specifically, the feed-side channel material may be a member having a continuous shape such as a net or a perforated film provided with protrusions, or may be a plurality of discontinuous members directly disposed on the separation membrane. The discontinuous members are provided such that their projected area ratio with respect to the separation membrane is more than 0 and less than 1. The feed-side channel material may be separable from the separation membrane or may be fixed to the separation membrane.

The material of the feed-side channel material is not particularly limited, and may be either the same as or different from the material of the separation membrane.

When the thickness of the feed-side channel material is large, the pressure loss decreases, but when an element is formed including such a feed-side channel material, the membrane area that can be packed in a pressure vessel decreases. When the thickness thereof is small, the pressure loss in the channel increases, and the separation performance or the permeation performance deteriorates. Considering the balance among performances or the operation cost, the thickness of the feed-side channel material is preferably 200 to 1000 $\mu$m, and more preferably 300 to 900 um. The thickness of the feed-side channel material can be directly measured with a commercially available thickness measuring instrument, or can be measured by analyzing an image taken using a microscope.

When the feed-side channel material is a net, the net is formed of a plurality of yarns. The plurality of yarns intersect with each other at intersection, and the thickness is the largest at the intersection portion. The diameters of the yarns constituting the net may be constant in a length direction of the yarns, or may be uniformly increased or decreased in the length direction, or may be a form in which the increase and decrease in diameter are repeated. When the diameters of the yarns are repeatedly increased and decreased in the length direction, it is preferable that a plurality of yarns intersect with each other at a position where the diameter of the yarn is the largest. By intersecting a plurality of yarns at the point where the diameter of the yarn is the largest, the pressure loss in the feed-side channel can be reduced.

The diameters of the plurality of intersecting yarns may be either the same or different. When the diameters of the plurality of intersecting yarns are different, if the thickness is constant, yarns with a small diameter have a large effect of reducing the pressure loss, and yarns with a large diameter have a large turbulence effect of disturbing the flow.

From the balance between the pressure loss and the turbulence effect, as for the diameter of the cross section of the yarn constituting the net, the ratio of (the diameter of the smallest part)/(the diameter of the largest part) is preferably 0.1 or more and 0.7 or less, and more preferably 0.3 or more and 0.6 or less.

If the inclination angle of the yarns constituting the net is parallel to the flowing direction of the feed liquid, the pressure loss can be lowered, but the concentration polarization reduction effect decreases. On the other hand, if the inclination angle is near a direction perpendicular to the flowing direction, the pressure loss increases, but the concentration polarization reduction effect can be increased. From the balance between the pressure loss and the concentration polarization reduction effect, the inclination angle of the yarns is preferably -60° or more and 60° or less with respect to an average flow angle of the feed liquid. Here, the average flow angle is an average value of flow angles in one separation membrane body.

The larger the interval between the intersections at which plurality of yarns intersect with each other, the smaller the pressure loss; whereas the smaller the interval, the larger the pressure loss. In terms of their balance, the intersection interval is preferably from 1.0 mm or more and 10 mm or less, more preferably 1.1 mm or more and 8 mm or less, and still more preferably 1.2 mm or more and 5 mm or less.

The cross sectional form of the yarns constituting the net is not particularly limited, and an ellipse, a circle, a triangle, a square, an irregular shape, or the like can be employed. It is preferable that the area of a portion where the net and the separating membrane surface are in contact is small because deterioration of the separation membrane performance due to rubbing between the separating membrane surface and the net can be inhibited, a flow dead zone can be reduced, and the concentration polarization can be inhibited. The projected area ratio of the portion where the net and the separating membrane surface are in contact with respect to the separation membrane is preferably 0.01 or more and 0.25 or less, and more preferably 0.02 or more and 0.2 or less.

The material of the yarns constituting the net is not particularly limited as long as it can maintain the rigidity as the feed-side channel material and does not damage the surface of the separation membrane. The material may be either the same as or be different from that of the separation membrane, and polyethylene, polypropylene, polylactic acid, an ethylene-vinyl acetate copolymer, polyester, polyurethane, a thermosetting elastomer, etc. can be suitably used.

The permeate-side channel material is disposed such that this material is sandwiched between the permeate-side faces of two facing separation membranes, and plays a role of forming a permeate-side channel that guides a permeate liquid that has permeated the separation membranes to the holes of the perforated central tube.

The permeate-side channel material preferably has a cross-sectional area ratio of 0.30 to 0.75, and more preferably 0.40 to 0.60 from the viewpoint of reducing the flow resistance of the permeate-side channel, inhibiting sinking of the separation membrane into the permeated liquid channel even under pressure filtration, and stably forming the channel. The type of the permeate-side channel material is not limited, and a weft knitted fabric such as tricot, a sheet in which protrusions are arranged on a porous sheet such as a nonwoven fabric, an irregularity-processed sheet obtained by subjecting a film or a nonwoven fabric to irregularity processing, or the like can be used.

The cross-sectional area ratio is defined, in a cross section obtained by cutting the permeate-side channel material such that the cutting line passes through a protrusion of the permeate-side channel material along the direction parallel to the longitudinal direction of the perforated central tube of the separation membrane element, by the ratio of the cross-sectional area of the permeate-side channel material occupied between the center of a certain protrusion and the center of an adjacent protrusion to the product of the distance between the center of the certain protrusion and the center of the adjacent protrusion and the height (thickness) of the permeate-side channel material. The cross-sectional area ratio can be calculated as an average value at 30 arbitrary positions using, for example, a high-precision shape analysis system KS-1100 manufactured by KEYENCE Corp.

When the thickness of the permeate-side channel material is large, the pressure loss can be reduced, but the membrane area that can be packed in the vessel of the separation membrane element decreases. When the thickness of the permeate-side channel material is small, the membrane area that can be packed in the separation membrane element increases, but the pressure loss increases. In terms of their balance, the thickness of the permeate-side channel material is preferably 0.1 mm to 0.5 mm, and more preferably 0.2 mm to 0.4 mm.

The thickness of the permeate-side channel material can be directly measured with a commercially available thickness measuring instrument.

The material of the permeate-side channel material may be any material as long as it can be easily wound around the perforated central tube, and the compressive elasticity modulus of the permeate-side channel is preferably 0.1 to 5 GPa. When the compressive elasticity modulus is within this range, the permeate-side channel material can be easily wound around the perforated central tube. Specifically, polyester, polyethylene, polypropylene, and the like are suitably used.

The compressive elasticity modulus of the permeate-side channel material can be measured by performing a compression test using a precision universal testing machine and preparing a stress-strain curve.

The perforated central tube is just required to be configured such that a permeate liquid flows therein, and the material and form thereof are not particularly limited. When the diameter of the perforated central tube is large, the membrane area that can be packed in the separation membrane element is reduced, whereas when the diameter is small, the flow resistance when a permeate liquid flows inside the perforated central tube increases. The diameter of the perforated central tube is appropriately designed according to the flow rate of the permeate liquid, and is preferably 10 to 50 mm, and more preferably 15 to 40 mm. As the perforated central tube, for example, a cylindrical member having a side face provided with a plurality of holes is used.

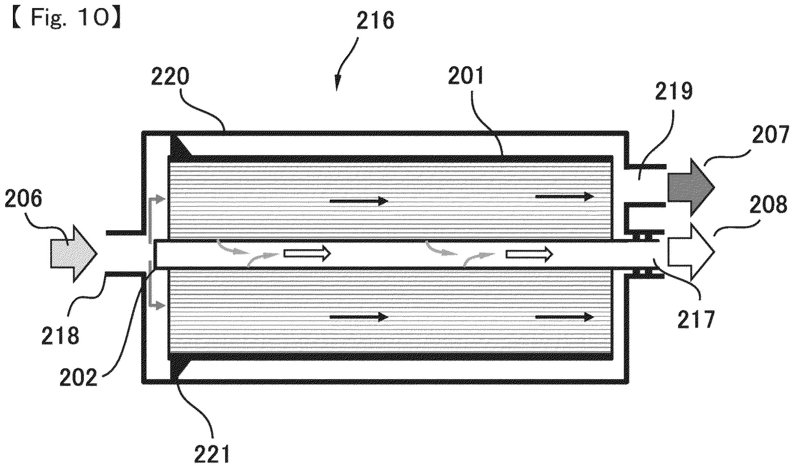

As illustrated in Fig. 10, a brine seal (221), which is a sealing member for preventing a feed liquid (206) and a concentrated liquid (207) from being mixed, is attached to an I-type separation membrane element, and the separation membrane element is enclosed in a pressure vessel (220) to form a separation membrane module.

<L-Type, reverse L-type, and U-turn-type separation membrane elements>

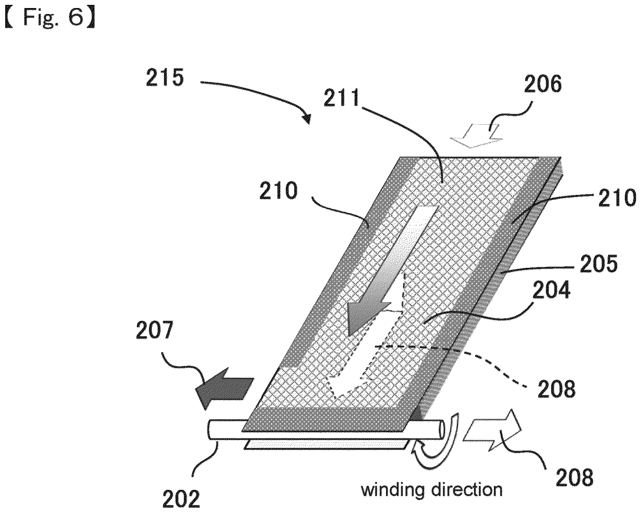

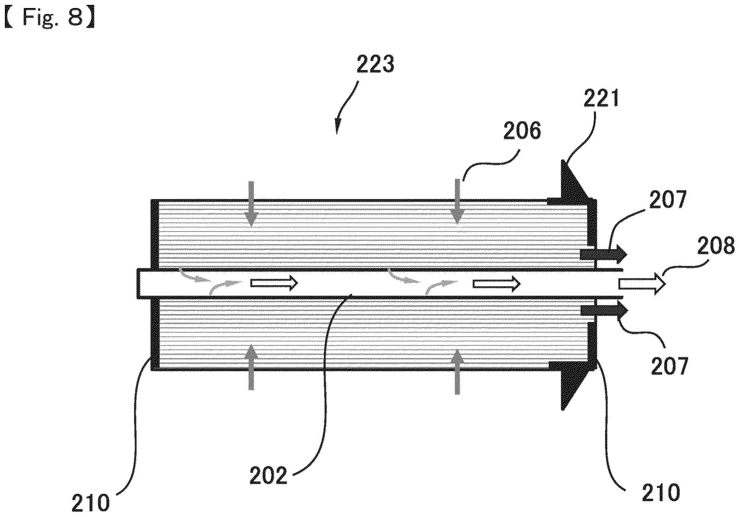

As illustrated in Fig. 8, in a separation membrane element (223) called L-type, a feed liquid (206) flows in from the outer peripheral part of the element, and a concentrated liquid (207) is discharged from one end face. The L-type separation membrane element (223) has a structure in which an L-type separation membrane body (215) illustrated in Fig. 6 is wound around a perforated central tube (202).

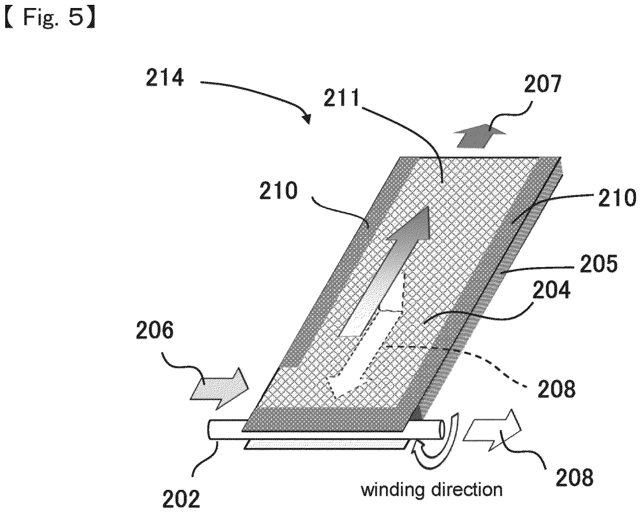

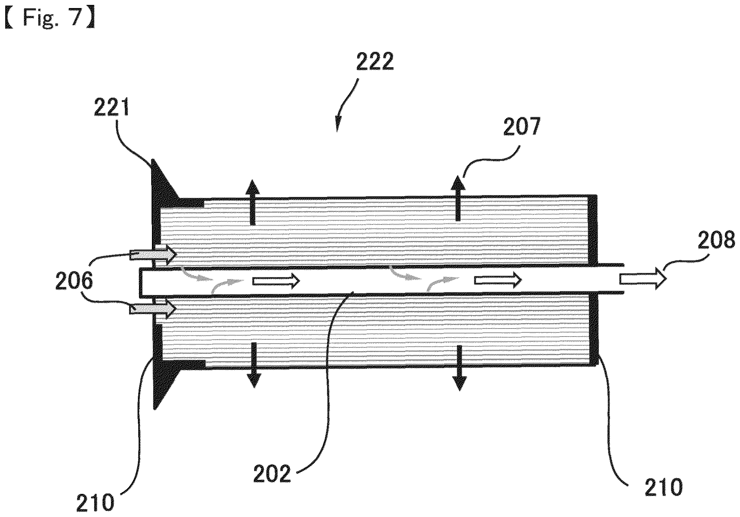

A separation membrane element (222) called reverse L-type is a separation membrane element having a structure in which a liquid flow is inverted from that of the L-type as illustrated in Fig. 7, and a feed liquid (206) flows in from one end face of the element, and a concentrated liquid (207) is discharged from an outer peripheral surface of the element. The reverse L-type separation membrane element (222) has a structure in which a reverse L-type separation membrane body (214) illustrated in Fig. 5 is wound around a perforated central tube (202).

The L-type or reverse L-type separation membrane element that performs an operation in which the recovery rate, which is the ratio of the permeate liquid to the feed liquid, is constant has the following advantages. That is, in the separation membrane element in which the ratio L/W of the length W of the separation membrane body in the longitudinal direction of the perforated central tube to the length L in the direction perpendicular to the longitudinal direction of the perforated central tube is 2.5 or more, the flow rate of the feed liquid passing through the feed-side channel in the separation membrane element is increased in comparison with the I-type separation membrane element with the same ratio L/W. Therefore, in the present invention, the L-type and reverse L-type separation membrane elements having an L/W of 2.5 or more can inhibit concentration polarization of a membrane surface even in a high recovery rate operation, and can be operated more stably. The channel materials to be used for the L-type and reverse L-type separation membrane elements may be the same as the channel material to be used for the I-type separation membrane element.

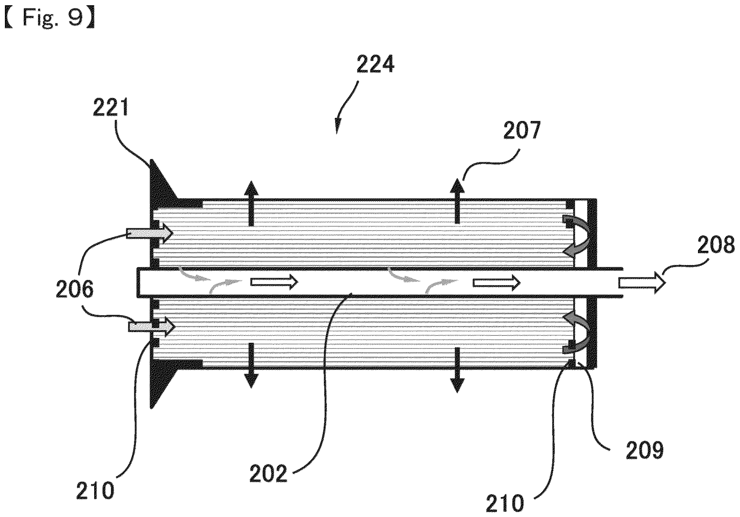

To further increase the flow rate of the feed liquid, a U-turn-type separation membrane element, which is a separation membrane element having two types of separation membrane bodies in one separation membrane element, may be used. One example of the U-turn-type separation membrane element is an I-type-reverse L-type separation membrane element (224) in which an I-type separation membrane body and a reverse L-type separation membrane body are combined as illustrated in Fig. 9. The I-type-reverse L-type separation membrane element (224) is a separation membrane element having a structure in which an I-type separation membrane body (213) in which a feed liquid (206) flows in from one end face in a longitudinal direction of a perforated central tube (202) and is discharged from the other end face as illustrated in Fig. 4 and a reverse L-type separation membrane body (214) in which a feed liquid (206) flows in from one end face in the longitudinal direction of the perforated central tube (202) and is discharged from an outer peripheral surface as illustrated in Fig. 5 are wound simultaneously. To the end face with an opening of the reverse L-type separation membrane body (214) in the I-type-reverse L-type separation membrane element (224), a U-turn cap (209) for U-turning the flow as illustrated in Fig. 9 is attached. As a result, since the feed liquid is to pass through the I-type separation membrane body (213) and then is fed to the reverse L-type separation membrane body (214), it is possible to form a structure capable of increasing the flow rate of the feed liquid (206) in which structure a so-called tree-type arrangement is simulated by one separation membrane element. By appropriately designing the ratio of the I-type separation membrane body (213) to the reverse L-type separation membrane body (214) and the above-described L/W, the I-type-reverse L-type separation membrane element (224) can increase the flow rate of a feed liquid to about 5 to 20 times that of a conventional I-type separation membrane element (201), and can be operated more stably even at a high recovery rate. The channel material to be used for the I-type-reverse L-type separation membrane element (224) may be the same as the channel material to be used for the I-type separation membrane element (201).

The L-type, reverse L-type, and U-turn-type separation membrane elements may be collectively called "high flow rate type separation membrane elements".

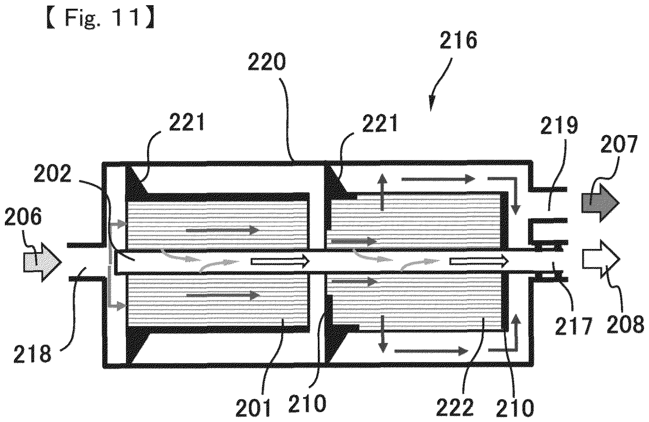

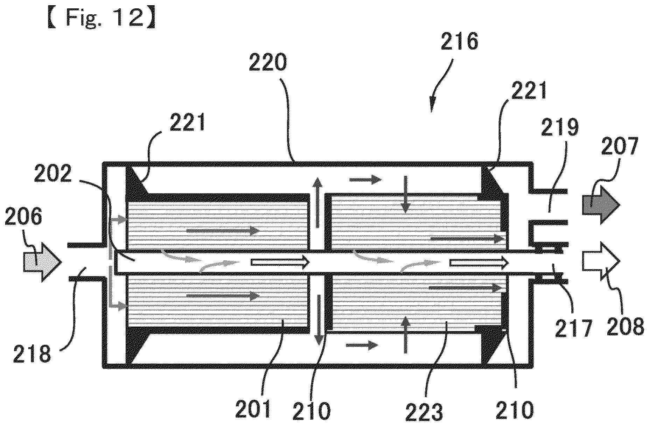

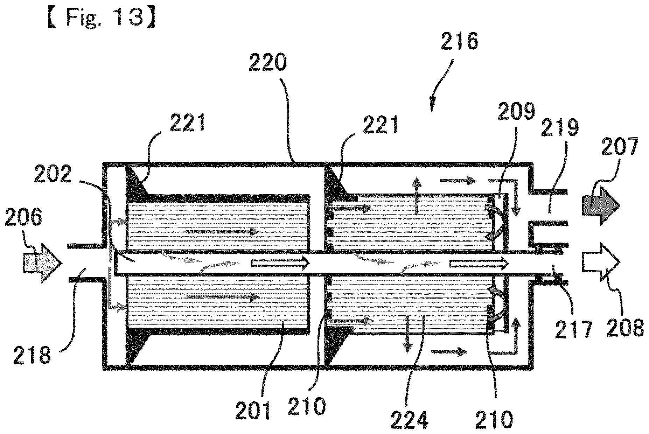

Next, a separation membrane module (216) in which an I-type separation membrane element (201) and a reverse L-type separation membrane element (222) are sealed in series in a pressure vessel (220) is illustrated in Fig. 11, a separation membrane module (216) in which an I-type separation membrane element (201) and an L-type separation membrane element (223) are sealed in series in a pressure vessel (220) is illustrated in Fig. 12, and a separation membrane module (216) in which an I-type separation membrane element (201) and an I-type-reverse L-type separation membrane element (224) are sealed in series in a pressure vessel (220) is illustrated in Fig. 13. Since the L-type, reverse L-type, and I-type-reverse L-type separation membrane elements generally have a short length in the longitudinal direction of the perforated central tube, in the embodiments illustrated in Figs. 11 to 13, the membrane areas of the separation membrane modules are secured by connecting the perforated central tube of the I-type separation membrane element having a short length in the longitudinal direction of the perforated central tube in series with the perforated central tube of the L-type, reverse L-type, or I-type-reverse L-type separation membrane element.

(4) Configuration of solution treatment method in the present invention

First, the recovery rate of an electrolyte such as a salt, the urea removal rate, and the potassium ion removal rate in the entire process are determined from the following formulas.

Electrolyte recovery rate = [(electrolyte concentration of purified liquid (102)) × (flow rate of purified liquid (102))]/[electrolyte concentration of raw liquid (101)) × flow rate of raw liquid (101))] × 100 [%] Urea removal rate = [(urea concentration of raw liquid (101)) × (flow rate of raw liquid (101)) - urea concentration of purified liquid (102)) × (flow rate of purified liquid (102))]/[urea concentration of raw liquid (101)) × (flow rate of raw liquid (101))] × 100 [%] Potassium ion removal rate = [(potassium ion concentration of raw liquid (101)) × (flow rate of raw liquid (101)) - (potassium ion concentration of purified liquid (102)) × (flow rate of purified liquid (102))] / [(potassium ion concentration of raw liquid (101)) × (flow rate of raw liquid (101))] × 100 [%]

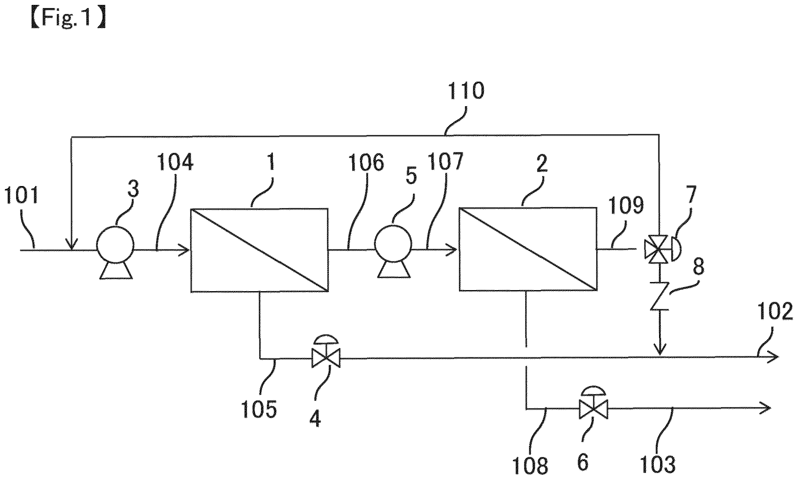

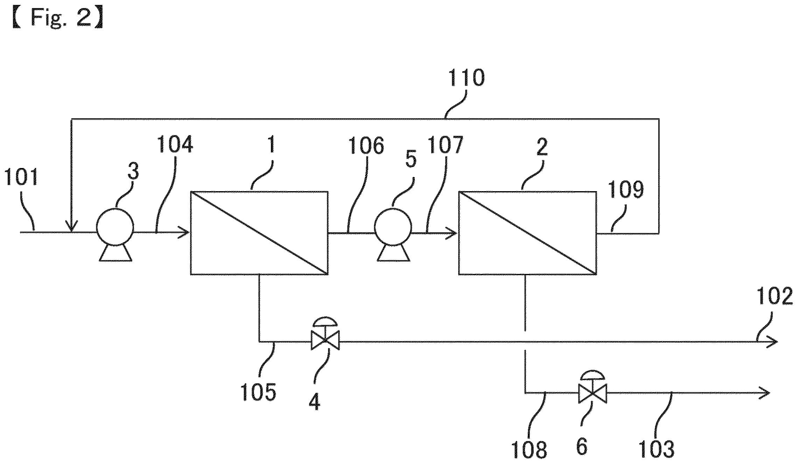

The numbers in parentheses in the above formulas mean the reference signs in Figs. 1 and 2.

As in the formulas given above, the electrolyte recovery rate, the urea removal rate, and the potassium ion removal rate as the entire process are defined not by a removal rate defined by a concentration but by the amount of substance including a flow rate ratio. This is because, for example, when the present membrane process is used for a regeneration treatment of a waste dialysate, the amount of water and the electrolyte concentration are adjusted by adding an adjustment solution in the subsequent stage to form a regenerated dialysate, so that the final concentration of the regenerated dialysate differs from the final concentration of the purified liquid.

In the solution treatment method of the present invention, a raw liquid is separated into a purified liquid containing water and an electrolyte and a discharge liquid containing a low-molecular-weight nonelectrolyte molecule using separation membranes 1 and 2 having the characteristics described above. At this time, the permeate liquid from the separation membrane 1 is fed to the separation membrane 2 and subjected to a separation treatment, and (i) a part of the permeate liquid from the separation membrane 2 is mixed with a raw liquid and then fed to the separation membrane 1, and the whole or a part of the remaining permeate liquid from the separation membrane 2 is mixed with the concentrated liquid from the separation membrane 1 to afford a purified liquid, or (ii) the whole of the permeate liquid from the separation membrane 2 is mixed with the raw liquid and then fed to the separation membrane 1, and the concentrated liquid from the separation membrane 1 is obtained as a purified liquid.

That is, a solution obtained by mixing the permeate liquid from the separation membrane 2 and the raw liquid is pressurized and treated with the separation membrane 1 having a sodium chloride removal rate of 90% or more and having a urea removal rate lower than that of the separation membrane 2 by 40% point or more, thereby affording the permeate liquid from the separation membrane 1 and the concentrated liquid from the separation membrane 1. At this time, the recovery rate, which is the ratio of the amount of the solution fed to the separation membrane 1 and the amount of the solution having permeated the separation membrane 1, is preferably 75% or more. By adopting such a recovery rate, the selective separation performance of the separation membrane 1 can be further exhibited. Since the separation membrane 1 has removal rates as described above, the permeate liquid from the separation membrane 1 contains a large amount of low-molecular-weight nonelectrolyte molecule such as urea, and does not contain much electrolyte. On the other hand, the concentrated liquid from the separation membrane 1 contains both an electrolyte and a low-molecular-weight nonelectrolyte molecule, but since the separation membrane 1 has a higher electrolyte removal rate than the urea removal rate, the concentrated liquid contains the electrolyte more than the low-molecular-weight nonelectrolyte molecule.

Further, the permeate liquid from the separation membrane 1 is treated with the separation membrane 2 having a urea removal rate higher than that of the separation membrane 1 by 40% point or more to afford a concentrated liquid from the separation membrane 2 and a permeate liquid from the separation membrane 2. At this time, the recovery rate, which is the ratio of the amount of the solution fed to the separation membrane 2 and the amount of the solution having permeated the separation membrane 1, is set according to the water recovery rate of the entire process, but since the permeate liquid from the separation membrane 2 is utilized for circulation to the feed liquid to the separation membrane 1, the recovery rate of the separation membrane 2 is preferably 50% or more. Since the separation membrane 2 exhibits the removal rates as described above, the permeate liquid from the separation membrane 2 contains an electrolyte and a low-molecular-weight nonelectrolyte molecule such as urea both only in a trace amount, and the concentrated liquid from the separation membrane 2 contains a low-molecular-weight nonelectrolyte molecule such as urea in a large amount.

Finally, the concentrated liquid from the separation membrane 1 is taken out while being mixed with the permeate liquid from the separation membrane 2, as necessary, to afford a purified liquid containing only a low-molecular-weight nonelectrolyte molecule such as urea selectively separated.

Embodiments of the solution treatment method of the present invention are shown in Figs. 1 and 2. Here, a module having the separation membrane 1 described above is denoted as separation membrane module (1), and a module having the separation membrane 2 described above is denoted as separation membrane module (2). In addition, in the following, for the sake of convenience, a case where a waste dialysate after being subjected to artificial dialysis treatment is treated as a raw liquid will be described, but the "urea" can be read as a "low-molecular-weight nonelectrolyte molecule".

In the solution treatment method of the present invention shown in Fig. 1, as described above, the urea that has permeated through the separation membrane module (1) is treated with the separation membrane module (2), taken out as a concentrated liquid (108) from the separation membrane module (2), and removed. Therefore, the removal rate of urea as a whole cannot be increased unless the amount of the permeate liquid (106) from the separation membrane module (1) is increased. That is, to increase the urea removal rate in the process, it is necessary to increase the recovery rate in the separation membrane module (1), but since the total component concentration in a waste dialysate, namely, the raw liquid (101) is generally as high as 10000 mg/L or more, the pressure applied to the separation membrane module (1) also needs to be increased in order to increase the recovery rate. For example, when the NaCl concentration in the feed liquid (104) to the separation membrane module (1) is 10000 mg/L, in the case where the separation membrane module (1) is operated at a recovery rate of 90%, the NaCl concentration in the concentrated liquid (105) from the separation membrane module (1) is about 10 times the concentration of the feed liquid, and the osmotic pressure difference is about 8.8 MPa. On the other hand, in the solution treatment method shown in Fig. 1, a part of the permeate liquid (109) from the separation membrane module (2) is mixed with the raw liquid (101) fed to the separation membrane module (1) and circulated. In a solution treatment method without the circulation of the permeate liquid (109) from the separation membrane module (2), there is a problem that the NaCl concentration in the concentrated liquid (105) from the separation membrane module (1) becomes high. However, by adopting the solution treatment method of the present invention as shown in Fig. 1, the NaCl concentration in the feed liquid (104) to the separation membrane module (1) can be lowered, and accordingly, the NaCl concentration in the concentrated liquid (105) from the separation membrane module (1) can be kept low even when the separation membrane module (1) is of high recovery. For example, when a raw liquid (101) having an NaCl concentration of 10000 mg/L is treated, if the NaCl concentration of the feed liquid (104) to the separation membrane module (1) can be reduced to 2000 mg/L by circulating the permeate liquid (109) from the separation membrane module (2), the difference in osmotic pressure between the concentrated liquid (105) and the permeate liquid (106) in the separation membrane module (1) is about 1.7 MPa even if the recovery rate of the separation membrane module (1) is 90%. As described above, in accordance with the present invention, it is possible to perform the treatment at a lower pressure as compared with the solution treatment method in which the circulation of the permeate liquid (109) from the separation membrane module (2) is not performed. If the treatment can be performed at a low pressure, it is unnecessary to use a high-pressure pump, a high-pressure-proof flow channel, and a pressure vessel, so that reduction in device cost or reduction in noise is expected. In particular, in consideration of the operating pressure in the waste dialysate treatment, the amount of the circulation (110) relative to the amount of the permeate liquid (109) from the separation membrane module (2) is preferably 0.6 or more. In addition, for adjustment of the concentration of the purified liquid (102) or other purpose, a part of the permeate liquid (109) from the separation membrane module (2) may be taken out, or pure water or a solution may be added to the purified liquid (102).

The solution treatment method shown in Fig. 2 is basically the same as the mode shown in Fig. 1, but all the permeate liquid (109) from the separation membrane module (2) is circulated by mixing it with the raw liquid (101). All the permeate liquid (109) from the separation membrane module (2) may be circulated by feeding it to the separation membrane module (1) as described above. This case is advantageous in that the osmotic pressure difference in the separation membrane module (1) can be significantly reduced. In addition, the valve operation at a branch portion of the permeate liquid (109), which is necessary in the embodiment shown in Fig. 1, is also unnecessary, so that both the device configuration and the process can be simplified.

(5) Dialysis system including waste dialysate treatment unit

To perform a waste dialysate treatment in accordance with the present invention, it is effective to incorporate a solution treatment unit in which lines are constituted in a flow as described in (4) above and valves and pumps are arranged at appropriate positions as a waste dialysate treatment unit into a dialysis system together with a dialyzer. That is, it is effective to combine a waste dialysate treatment unit (301) with a dialyzer (302) as shown in Fig. 14. In this way, it is possible to perform dialysis treatment while treating a waste dialysate (307) online. In the waste dialysate treatment unit (301), minute water and electrolytes are discharged as a discharge liquid (103). Therefore, as shown in Fig. 14, an adjustment solution (305) is added to a purified liquid (102) from a waste dialysate treatment unit (301) to afford a regenerated dialysate (304).

In addition, it is also possible to combine the waste dialysate treatment unit of the present application with a tank, treat a waste dialysate offline, thereby adjust the electrolyte concentration, and then subject the waste dialysate to dialysis treatment.

EXAMPLES

Hereinafter, the present invention will be described in more detail with reference to Examples, but the present invention is not limited by the Examples at all.

(Measurement of separation membrane performance)

For PA membrane A, PA membrane B, PA membrane C, PA membrane F, which are polyamide membranes, and CA membrane, which is a cellulose acetate membrane, the NaCl removal rate and the urea removal rate were measured at a temperature of 36°C and a pressure of 1.2 MPa for a solution 1 at pH 7 in which 10000 mg/L of sodium chloride (NaCl) and 250 mg/L of urea were mixed.

For PA membrane D, PA membrane E, and PA membrane G, which are polyamide membranes, the NaCl removal rate and the urea removal rate were measured at a temperature of 36°C and a pressure of 1.8 MPa for a solution 2 at pH 7 in which 1000 mg/L of sodium chloride (NaCl) and 700 mg/L of urea were mixed.

It is noted that the removal rates referred to here are each defined by a reduction rate of the component concentration in the permeated solution relative to the component concentration in the feed liquid for each membrane.

(Positron annihilation lifetime spectroscopy by positron beam method)

Positron annihilation lifetime measurement of a separation functional layer in each example was performed using a positron beam method as follows. Specifically, the separation functional layer was dried at room temperature under reduced pressure, and cut into a 1.5 cm × 1.5 cm square, and thus an inspection sample was prepared. In a positron annihilation lifetime measurement device for thin membrane equipped with a positron beam generator (this device is described in detail in, for example, Radiation Physics and Chemistry, 58, 603, Pergamon (2000)), the inspection sample was measured at a total count of 5,000,000 by a scintillation counter made of barium difluoride through using a photomultiplier tube at a beam intensity of 1 keV at room temperature in vacuum, and analyzed by POSI TRONFIT. An average pore diameter was calculated on the basis of an average positron annihilation lifetime $\tau$ of a fourth component obtained by the analysis.

(Preparation of microporous support membrane (porous support layer))

A 16.0% by weight DMF (dimethylformamide) solution of polysulfone (PSf) was cast at room temperature (25°C) on a polyester nonwoven fabric (air permeability: 2.0 cc/cm$^2$/sec) to a thickness of 200 pm, and immediately immersed in pure water and allowed to stand for 5 minutes, and thus a support membrane was prepared.

(Preparation of PA membrane A)

A 1.5% by weight aqueous solution of m-phenylenediamine was prepared. The support membrane obtained by the above operation was immersed in the above aqueous solution for 2 minutes, the support membrane was slowly pulled up in a vertical direction, blown with nitrogen from an air nozzle to remove excess aqueous solution from a surface of the support membrane, then applied with a 25°C decane solution containing 0.065% by weight of trimesoyl chloride (TMC) in a booth maintained at 25°C such that the surface was completely wetted, and was allowed to stand for 60 seconds. Thus, a reverse osmosis membrane was obtained. The performance of the obtained reverse osmosis membrane was an NaCl removal rate of 97% and a urea removal rate of 18%. The pore diameter measured using a positron annihilation lifetime spectroscopy was 7.2 Å.

(Preparation of PA membrane B)

A 1.8% by weight aqueous solution of m-phenylenediamine was prepared. The support membrane obtained by the above operation was immersed in the above aqueous solution for 2 minutes, the support membrane was slowly pulled up in a vertical direction, blown with nitrogen from an air nozzle to remove excess aqueous solution from a surface of the support membrane, then applied with a 25°C decane solution containing 0.06% by weight of trimesoyl chloride (TMC) in a booth maintained at 25°C such that the surface was completely wetted, and was allowed to stand for 60 seconds. Thus, a reverse osmosis membrane was obtained. The performance of the obtained reverse osmosis membrane was an NaCl removal rate of 98% and a urea removal rate of 25%. The pore diameter measured using a positron annihilation lifetime spectroscopy was 7.0 Å.

(Preparation of PA membrane C)

A 0.2% by weight aqueous solution of piperazine was prepared. The support membrane obtained by the above operation was immersed in the above aqueous solution for 2 minutes, the support membrane was slowly pulled up in a vertical direction, blown with nitrogen from an air nozzle to remove excess aqueous solution from a surface of the support membrane, then applied with a 45°C decane solution containing 0.17% by weight of trimesoyl chloride (TMC) in a booth maintained at 45°C such that the surface was completely wetted, and was allowed to stand for 1 minute. Next, to remove excess solution from the membrane, the membrane was held vertically for 1 minute to perform liquid draining, and then the membrane was dried by blowing a gas at 25°C using a blower. After the drying, the membrane was immediately washed with water and stored at room temperature, affording a nanofiltration membrane. The performance of the obtained nanofiltration membrane was an NaCl removal rate of 93.7% and a urea removal rate of 16%. The pore diameter measured using a positron annihilation lifetime spectroscopy was 9.0 Å.

(Preparation of PA membrane D)

A 6.0% by weight aqueous solution of m-phenylenediamine was prepared. The support membrane obtained by the above operation was immersed in the above aqueous solution for 2 minutes, the support membrane was slowly pulled up in a vertical direction, blown with nitrogen from an air nozzle to remove excessive aqueous solution from a surface of the support membrane, then applied with a 45°C decane solution containing 0.17% by weight of trimesoyl chloride (TMC) in a booth maintained at 45°C such that the surface was completely wetted, and was allowed to stand for 10 seconds. The support membrane was placed in an oven at 140°C and heated for 30 seconds while water vapor at 100°C was supplied from a nozzle provided on a back surface side of the membrane, affording a reverse osmosis membrane. The performance of the obtained reverse osmosis membrane was an NaCl removal rate of 99.6% and a urea removal rate of 90%. The pore diameter measured using a positron annihilation lifetime spectroscopy was 5.1 Å.

(Preparation of PA membrane E)

A 5.5% by weight aqueous solution of m-phenylenediamine was prepared. The support membrane obtained by the above operation was immersed in the above aqueous solution for 2 minutes, the support membrane was slowly pulled up in a vertical direction, blown with nitrogen from an air nozzle to remove excessive aqueous solution from a surface of the support membrane, then applied with a 45°C decane solution containing 0.15% by weight of trimesoyl chloride (TMC) in a booth maintained at 45°C such that the surface was completely wetted, and was allowed to stand for 10 seconds. The support membrane was placed in an oven at 140°C and heated for 30 seconds while water vapor at 100°C was supplied from a nozzle provided on a back surface side of the membrane, affording a reverse osmosis membrane. The performance of the obtained reverse osmosis membrane was an NaCl removal rate of 99.1% and a urea removal rate of 85%. The pore diameter measured using a positron annihilation lifetime spectroscopy was 6.0 Å.

(Preparation of PA membrane F)

A 2.0% by weight aqueous solution of m-phenylenediamine was prepared. The support membrane obtained by the above operation was immersed in the above aqueous solution for 2 minutes, the support membrane was slowly pulled up in a vertical direction, blown with nitrogen from an air nozzle to remove excessive aqueous solution from a surface of the support membrane, then applied with a 25°C decane solution containing 0.12% by weight of trimesoyl chloride (TMC) in a booth maintained at 25°C such that the surface was completely wetted, and was allowed to stand for 40 seconds. The support membrane was placed in an oven at 140°C and heated for 30 seconds while water vapor at 100°C was supplied from a nozzle provided on a back surface side of the membrane, affording a reverse osmosis membrane. The performance of the obtained reverse osmosis membrane was an NaCl removal rate of 99.0% and a urea removal rate of 58%. The pore diameter measured using a positron annihilation lifetime spectroscopy was 6.8 Å.

(Preparation of PA membrane G)

An aqueous solution containing 1.8% by weight of m-phenylenediamine and 4.5% by weight of $\varepsilon$-caprolactam was prepared. The aqueous solution was applied to the support membrane obtained by the above operation, and then blown with nitrogen from an air nozzle to remove excessive aqueous solution from a surface of the support membrane. Then, a 25°C n-decane solution containing 0.06% by weight of trimesoyl chloride was applied to the support membrane such that the surface was completely wetted. Thereafter, excess solution was removed from the membrane by air blowing, the membrane was washed with hot water at 80°C, and then subjected to draining by air blowing, affording a reverse osmosis membrane. The performance of the obtained reverse osmosis membrane was an NaCl removal rate of 98.5% and a urea removal rate of 75%. The pore diameter measured using a positron annihilation lifetime spectroscopy was 7.2 Å.

(Preparation of cellulose acetate (CA) membrane)

A cast solution prepared by mixing 25% by weight of cellulose acetate, 45% by weight of acetone, and 30% by weight of formamide was cast on the support membrane obtained by the above operation, and was evaporated for 2 minutes, and then the support membrane was immersed in iced water. Next, the membrane was immersed in hot water at 90°C, affording a nanofiltration membrane. The performance of the obtained nanofiltration membrane was an NaCl removal rate of 93.0% and a urea removal rate of 15%. The pore diameter measured using a positron annihilation lifetime spectroscopy was 10 Å.

(Preparation of I-type separation membrane element)

A separation membrane was cut into six pieces, and each piece was folded with the feed side facing inward such that the inner peripheral end was a fold. Then, a net (thickness: 0.8 mm, pitch of fibers constituting the net (hereinafter, briefly referred to as pitch): 5 mm × 5 mm, fiber diameter: 380 um, projected area ratio of a portion where the net and the separation membrane surface were in contact with respect to the separation membrane (hereinafter, briefly referred to as projected area ratio): 0.15) was used as a feed-side channel material, and was disposed such that the inclination angle of yarns constituting the net was 45° with respect to the winding direction. As a permeate-side channel material, tricot having a uniform thickness (thickness: 280 um) was prepared, and cut into six pieces. From these pieces, six separation membrane bodies having a length of 850 mm and a width of 930 mm, 465 mm, or 230 mm were prepared. The permeate-side channel material was disposed on the permeate-side face of the separation membrane, an adhesive was applied to the permeate-side channel material such that the inner peripheral end was opened, and the channel material was spirally wound around a perforated central tube (length: 1020 mm, 510 mm, or 280 mm, diameter: 30 mm, number of holes: 40 or 20 holes $\times$ 1 linear row) made of ABS (acrylonitrile-butadiene-styrene). After winding, a film was wound around the outer periphery and fixed with a tape, followed by edge cutting, attachment of an end plate, and filament winding, and thus an I-type separation membrane element having an effective membrane area of 8 m$^2$, 4 m$^2$, or 2 m$^2$ was prepared.

(Preparation of L-type and reverse L-type separation membrane elements)

A separation membrane was cut into six pieces, and each piece was folded with the feed side facing inward such that the inner peripheral end was a fold. Then, a net (thickness: 0.8 mm, pitch: 5 mm $\times$ 5 mm, fiber diameter: 380 um, projected area ratio: 0.15) was used as a feed-side channel material, and was disposed such that the inclination angle of yarns constituting the net was 45° with respect to the winding direction. As a permeate-side channel material, tricot having a uniform thickness (thickness: 280 um) was prepared, and cut into six pieces. From these pieces, six separation membrane bodies having a length of 850 mm and a width of 230 mm were prepared. The ratio L/W of the length W of each of the separation membrane bodies in the longitudinal direction of the perforated central tube to the length L in a direction perpendicular to the longitudinal direction of the perforated central tube is 3.7. The permeate-side channel material was disposed on the permeate-side face of the separation membrane, an adhesive was applied to the permeate-side channel material such that the inner peripheral end was opened, and the channel material was spirally wound around a perforated central tube (length: 280 mm, diameter: 30 mm, number of holes: 12 holes $\times$ 1 linear row) made of ABS (acrylonitrile-butadiene-styrene). After the winding, a film with holes was wound around the outer periphery and fixed with a tape, and then edge cutting was performed. Thereafter, an adhesive was applied to one end face in the longitudinal direction of the perforated central tube to perform sealing. Further, an adhesive was applied such that 20% of the inner peripheral portion of the end face opposite from the sealed side was opened, and thus L-type and reverse L-type separation membrane elements having an effective membrane area of 2 m$^2$ were prepared. In the L-type and reverse L-type separation membrane elements, since the above-described ratio L/W is 3.7, the length L is 1/4 of the length of the I-type separation membrane element having an effective membrane area of 8 m$^2$, and accordingly, the effective membrane area is also 1/4 of that of the I-type separation membrane element having an effective membrane area of 8 m$^2$. In addition, this separation membrane element serves as an L-type separation membrane element when a feed liquid is fed from the outer peripheral part, and serves as a reverse L-type separation membrane element when a feed liquid is fed from one end face of the separation membrane element.

(Preparation of U-turn-type separation membrane element)

A separation membrane was cut into six pieces, and each piece was folded with the feed side facing inward such that the inner peripheral end was a fold. Then, a net (thickness: 0.8 mm, pitch: 5 mm $\times$ 5 mm, fiber diameter: 380 um, projected area ratio: 0.15) was used as a feed-side channel material, and was disposed such that the inclination angle of yarns constituting the net was 45° with respect to the winding direction. Among these separation membranes sandwiching the feed-side channel material, the outer peripheral ends of the feed-side channel materials of three separation membranes were bonded such that an I-type separation membrane body illustrated in Fig. 4 was formed, and the other three separation membranes were bonded on both end faces in the longitudinal direction of the perforated central tube such that a reverse L-type separation membrane body illustrated in Fig. 5 was formed. The opening ratio of the opening of one end face to the length of the separation membrane body was set to 20%. As a permeate-side channel material, tricot having a uniform thickness (thickness: 280 um) was prepared, and cut into six pieces. From these pieces, six separation membrane bodies having a length of 850 mm and a width of 230 mm were prepared. The ratio L/W of the length W of each of the separation membrane bodies in the longitudinal direction of the perforated central tube to the length L in a direction perpendicular to the longitudinal direction of the perforated central tube is 3.7. The permeate-side channel material was disposed on the permeate-side face of the separation membrane, an adhesive was applied to the permeate-side channel such that the inner peripheral end was opened, and the channel material was spirally wound around a perforated central tube (length: 280 mm, diameter: 30 mm, number of holes: 12 holes $\times$ 1 linear row) made of ABS (acrylonitrile-butadiene-styrene). After the winding, a film with holes was wound around the outer periphery and fixed with a tape, and then edge cutting was performed, and then an adhesive was applied near the outer periphery of both end faces in the longitudinal direction of the perforated central tube. Thereafter, a U-turn cap for U-turning a feed liquid flow was attached to the end face of the reverse L-type separation membrane body on the side with the opening, and thus a U-turn-type separation membrane element having an effective membrane area of 2 m$^2$ was prepared. In the U-turn-type separation membrane element, since the above-described L/W is 3.7, the length L is 1/4 of the length of the I-type separation membrane element having an effective membrane area of 8 m$^2$, and accordingly, the effective membrane area is also 1/4 of that of the I-type separation membrane element having an effective membrane area of 8 m$^2$.

(Raw liquid)

In Examples 1 to 10 and Comparative Examples 1 to 3, an artificial waste dialysate was used as a raw liquid. The components in the raw liquid were electrolyte: approximately 10000 mg/L (including 100 mg/L of potassium ion), and urea (molecular weight 60.06): 630 mg/L.

In Example 11, brackish water having a high boron concentration was used as a raw liquid. The components in the raw liquid were electrolyte: 1500 mg/L, and boron (in the form of boric acid) (molecular weight: 61.83): 1.5 mg/L.

(Measurement of process performance)

The electrolyte recovery rate, the urea removal rate, and the boron removal rate in the entire process were determined from the following formulas.

Electrolyte recovery rate = [(electrolyte concentration of purified liquid (102)) × (flow rate of purified liquid (102))]/[electrolyte concentration of raw liquid (101)) × flow rate of raw liquid (101)] × 100 [%] Urea removal rate = [(urea concentration of raw liquid (101)) × (flow rate of raw liquid (101)) - urea concentration of purified liquid (102) × (flow rate of purified liquid (102))]/[urea concentration of raw liquid (101)) × (flow rate of raw liquid (101))] × 100 [%] Potassium ion removal rate = [(potassium ion concentration of raw liquid (101)) × (flow rate of raw liquid (101)) - (potassium ion concentration of purified liquid (102)) × (flow rate of purified liquid (102))] / [(potassium ion concentration of raw liquid (101)) × (flow rate of raw liquid (101))] × 100 [%] Boron removal rate = [(boron concentration of raw liquid (101)) - boron concentration of purified liquid (102)) / [(boron concentration of raw liquid (101))] × 100 [%]

The numbers in parentheses in the above formulas mean the reference signs in Figs. 1 and 2.

Furthermore, the maximum operation pressure and the maximum pump flow rate in each module were also measured with a flow meter and a pressure gauge.

(Example 1)

A waste dialysate was used as the raw liquid. The PA membrane A obtained by the method as described above was use as the separation membrane 1, and the PA membrane D obtained by the method as described above was used as the separation membrane 2. As each of both separation membrane elements, one I-type separation membrane element having a width of 930 mm was prepared by the method described above.

To each of the separation membrane elements prepared were attached an end plate and a brine seal, and the separation membrane elements were each placed in a pressure vessel, affording one separation membrane module (1) and one separation membrane module (2). As shown in Fig. 2, pumps and valves were prepared, and piping was connected. When the valve was adjusted such that the total solution recovery rate was 97% and the urea removal rate was about 70%, and evaluation was performed under the conditions given in Table 1, the results were as shown in Table 1.

[Table 1]

[Table 1]

|  |  |  | Example 1 | Example 2 | Example 3 | Example 4 | Example 5 | Example 6 |
|---|---|---|---|---|---|---|---|---|
| Separation membrane 1 |  | Type | PA membrane A | PA membrane A | CA membrane | PA membrane A | PA membrane B | PA membrane C |
|  |  | Electrolyte removal rate [%] | 97.0 | 97.0 | 93.0 | 97.0 | 98.0 | 93.7 |
|  |  | Urea removal rate [%] | 18.0 | 18.0 | 15.0 | 18.0 | 25.0 | 16.0 |
|  |  | Module type | I-Type | I-Type | I-Type | I-Type | I-Type | I-Type |
|  |  | 2 Total membrane area [m$^2$] | 8.0 | 4.0 | 4.0 | 8.0 | 8.0 | 4.0 |
| Separation membrane 2 |  | Type | PA membrane D | PA membrane D | PA membrane D | PA membrane E | PA membrane D | PA membrane D |
|  |  | Electrolyte removal rate [%] | 99.6 | 99.6 | 99.6 | 99.1 | 99.6 | 99.6 |
|  |  | Urea removal rate [%] | 90.0 | 90.0 | 90.0 | 85.0 | 90.0 | 90.0 |
|  |  | Module type | I-Type | I-Type | I-Type | I-Type | I-Type | I-Type |
|  |  | 2 Total membrane area [m$^2$] | 8 | 4 | 4 | 8 | 8 | 4 |
|  |  | Permeate liquid circulation ratio | 1 | 0.80 | 0.85 | 0.85 | 0.85 | 0.85 |
| Solution treatment method |  |  | Fig. 2 | Fig. 1 | Fig. 1 | Fig. 1 | Fig. 1 | Fig. 1 |
| Raw liquid treatment amount [L/min] |  |  | 0.51 | 0.51 | 0.51 | 0.51 | 0.51 | 0.51 |
| Solution recovery rate [%] |  |  | 97.0 | 97.0 | 93.0 | 95.0 | 97.0 | 93.0 |

(continued)

| | | Example 1 | Example 2 | Example 3 | Example 4 | Example 5 | Example 6 |
|---|---|---|---|---|---|---|---|
| Operation results | Electrolyte recovery rate [%] | 92.7 | 91.7 | 83.7 | 90.9 | 93.8 | 85.0 |
| | Urea removal rate [%] | 70.1 | 69.9 | 70.2 | 69.8 | 70.0 | 70.3 |
| | Potassium ion removal rate [%] | 8.7 | 9.9 | 17 .3 | 10.5 | 7.6 | 17.9 |
| | Separation membrane module 1 Maximum operation pressure [MPa] | 0.89 | 2.57 | 1.55 | 1.97 | 2.45 | 1.61 |
| | Separation membrane module 2 Maximum operation pressure [MPa] | 3.29 | 3.52 | 3.20 | 2.65 | 2.67 | 3.18 |
| | Separation membrane module 1 Maximum pump flow rate [L/min] | 3.91 | 1.81 | 1.80 | 2.89 | 2.26 | 1.82 |
| | Separation membrane module 2 Maximum pump flow rate [L/min] | 3.42 | 1.64 | 1.56 | 2.67 | 2.08 | 1.58 |

(Example 2)

A waste dialysate was used as the raw liquid. One separation membrane module (1) and one separation membrane module (2) were prepared in the same manner as in Example 1 except that the width of the I-type separation membrane elements was 465 mm. Using these, pumps and valves were prepared, and piping was connected as shown in Fig. 1. When the valve was adjusted such that the total solution recovery rate was 97% and the urea removal rate was about 70%, and evaluation was performed under the conditions given in Table 1, the results were as shown in Table 1. The circulation ratio of the purified liquid was 0.80. As a result of the fact that a part of the permeate liquid from the separation membrane module (2) is recovered as a purified liquid, the amount of water to the separation membrane modules (1) and (2) is reduced as compared with Example 1, and a system can be established with a half membrane area of Example 1.

(Example 3)

A waste dialysate was used as the raw liquid. Separation membrane modules were prepared in the same manner as in Example 2 except that the separation membrane used for the separation membrane module (1) was the CA membrane. To each of the separation membrane elements prepared were attached an end plate and a brine seal, and the separation membrane elements were each placed in a pressure vessel, affording one separation membrane module (1) and one separation membrane module (2). As shown in Fig. 1, pumps and valves were prepared, and piping was connected. When the valve was adjusted such that the total solution recovery rate was 93% and the urea removal rate was about 70%, and evaluation was performed under the conditions given in Table 1, the results were as shown in Table 1. The circulation ratio of the permeate liquid from the separation membrane module (2) was 0.85.

(Example 4)

A waste dialysate was used as the raw liquid. Separation membrane modules were prepared in the same manner as in Example 1 except that the separation membrane used for the separation membrane module (2) was the PA membrane E. To each of the separation membrane elements prepared were attached an end plate and a brine seal, and the separation membrane elements were each placed in a pressure vessel, affording one separation membrane module (1) and one separation membrane module (2). As shown in Fig. 1, pumps and valves were prepared, and piping was connected. When the valve was adjusted such that the total solution recovery rate was 95% and the urea removal rate was about 70%, and evaluation was performed under the conditions given in Table 1, the results were as shown in Table 1. The circulation ratio of the permeate liquid from the separation membrane module (2) was 0.85.

(Example 5)

A waste dialysate was used as the raw liquid. Separation membrane modules were prepared in the same manner as in Example 1 except that the separation membrane used for the separation membrane module (1) was the PA membrane B. To each of the separation membrane elements prepared were attached an end plate and a brine seal, and the separation membrane elements were each placed in a pressure vessel, affording one separation membrane module (1) and one separation membrane module (2). As shown in Fig. 1, pumps and valves were prepared, and piping was connected. When the valve was adjusted such that the total solution recovery rate was 97% and the urea removal rate was about 70%, and evaluation was performed under the conditions given in Table 1, the results were as shown in Table 1. The circulation ratio of the permeate liquid from the separation membrane module (2) was 0.85.

(Example 6)

A waste dialysate was used as the raw liquid. Separation membrane modules were prepared in the same manner as in Example 2 except that the separation membrane used for the separation membrane module (1) was the PA membrane C. To each of the separation membrane elements prepared were attached an end plate and a brine seal, and the separation membrane elements were each placed in a pressure vessel, affording one separation membrane module (1) and one separation membrane module (2). As shown in Fig. 1, pumps and valves were prepared, and piping was connected. When the valve was adjusted such that the total solution recovery rate was 93% and the urea removal rate was about 70%, and evaluation was performed under the conditions given in Table 1, the results were as shown in Table 1. The circulation ratio of the permeate liquid from the separation membrane module (2) was 0.85.

(Example 7)

A waste dialysate was used as the raw liquid. Separation membrane modules the same as those in Example 2 were prepared. To each of the separation membrane elements prepared were attached an end plate and a brine seal, and the separation membrane elements were each placed in a pressure vessel, affording one separation membrane module (1) and one separation membrane module (2). As shown in Fig. 1, pumps and valves were prepared, and piping was connected. When the valve was adjusted such that the total solution recovery rate was 75% and the urea removal rate was about 70%, and evaluation was performed under the conditions given in Table 2, the results were as shown in Table 2. The circulation ratio of the permeate liquid from the separation membrane module (2) was 0.60.

[Table 2]

[Table 2]

| | | | Example 7 | Example 8 | Example 9 | Example 10 | Example 11 |
|---|---|---|---|---|---|---|---|
| Separation membrane 1 | | Type | PA membrane A | PA membrane A | PA membrane A | PA membrane A | PA membrane A |
| | | Electrolyte removal rate [%] | 97.0 | 97.0 | 97.0 | 97.0 | 97.0 |
| | | Urea removal rate [%] | 18.0 | 18.0 | 18.0 | 18.0 | 18.0 |
| | | Module type | I-Type | I-Type + reverse L-type | I-Type + L-type | I-Type + U-turn-type | I-Type |
| | | Total membrane area [m$^2$] | 4.0 | 4.0 | 4.0 | 4.0 | 4.0 |
| Separation membrane 2 | | Type | PA membrane D | PA membrane D | PA membrane D | PA membrane D | PA membrane D |
| | | Electrolyte removal rate [%] | 99.6 | 99.6 | 99.6 | 99.6 | 99. 6 |
| | | Urea removal rate [%] | 90.0 | 90.0 | 90.0 | 90.0 | 90.0 |
| | | Module type | I-Type | I-Type + reverse L-type | I-Type + L-type | I-Type + U-turn-type | I-Type |
| | | Total membrane area [m$^2$] | 4 | 4 | 4 | 4 | 4 |
| | | Permeate liquid circulation ratio | 0.60 | 0.80 | 0.80 | 0.80 | 0.80 |
| Solution treatment method | | | Fig. 1 | Fig. 1 | Fig. 1 | Fig. 1 | Fig. 1 |
| Raw liquid treatment amount [L/min] | | | 0.51 | 0.51 | 0.51 | 0.51 | 0.51 |
| Solution recovery rate [%] | | | 75.0 | 97.0 | 97.0 | 97.0 | 90.0 |

(continued)

| | | Example 7 | Example 8 | Example 9 | Example 10 | Example 11 |
|---|---|---|---|---|---|---|
| Operation results | Electrolyte recovery rate [%] | 93.3 | 91.9 | 91.7 | 92.4 | 89.3 |
| | Urea removal rate [%] (Example 11: boron removal rate) | 70.2 | 69.9 | 70.0 | 70.0 | 76.0 |
| | Potassium ion removal rate [%] | 7.8 | 9.3 | 9 . 6 | 8 . 8 | - |
| | Separation membrane module 1 Maximum operation pressure [MPa] | 2.19 | 2.37 | 2.44 | 2.19 | 0.62 |
| | Separation membrane module 2 Maximum operation pressure [MPa] | 1.62 | 3.31 | 3.35 | 3.26 | 3.18 |
| | Separation membrane module 1 Maximum pump flow rate [L/min] | 1.23 | 1.78 | 1.79 | 1.75 | 2.03 |
| | Separation membrane module 2 Maximum pump flow rate [L/min] | 1.03 | 1.60 | 1.62 | 1.57 | 1.93 |

(Example 8)

A waste dialysate was used as the raw liquid. Using the same separation membranes as those in Example 1, one I-type separation membrane element and one reverse L-type element each having a width of 230 mm were prepared as the separation membrane modules (1) and (2). To each of the separation membrane elements were attached an end plate and a brine seal, and the separation membrane elements were enclosed in a pressure vessel such that the reverse L-type separation membrane element was positioned at the subsequent stage as illustrated in Fig. 11. As shown in Fig. 1, pumps and valves were prepared, and piping was connected. When the valve was adjusted such that the total solution recovery rate was 97% and the urea removal rate was about 70%, and evaluation was performed under the conditions given in Table 2, the results were as shown in Table 2. The circulation ratio of the permeate liquid from the separation membrane module (2) was 0.80.

(Example 9)

A waste dialysate was used as the raw liquid. Using the same separation membranes as those in Example 1, one I-type separation membrane element and one L-type element each having a width of 230 mm were prepared as the separation membrane modules (1) and (2). To each of the separation membrane elements were attached an end plate and a brine seal, and the separation membrane elements were enclosed in a pressure vessel such that the L-type separation membrane element was positioned at the subsequent stage as illustrated in Fig. 12. As shown in Fig. 1, pumps and valves were prepared, and piping was connected. When the valve was adjusted such that the total solution recovery rate was 97% and the urea removal rate was about 70%, and evaluation was performed under the conditions given in Table 2, the results were as shown in Table 2. The circulation ratio of the permeate liquid from the separation membrane module (2) was 0.80.

(Example 10)

A waste dialysate was used as the raw liquid. Using the same separation membranes as those in Example 1, one I-type separation membrane element and one U-turn-type element each having a width of 230 mm were prepared as the separation membrane modules (1) and (2). To each of the separation membrane elements were attached an end plate and a brine seal, and the separation membrane elements were enclosed in a pressure vessel such that the U-turn-type separation membrane element was positioned at the subsequent stage as illustrated in Fig. 13. As shown in Fig. 1, pumps and valves were prepared, and piping was connected. When the valve was adjusted such that the total solution recovery rate was 97% and the urea removal rate was about 70%, and evaluation was performed under the conditions given in Table 2, the results were as shown in Table 2. The circulation ratio of the permeate liquid from the separation membrane module (2) was 0.80.

(Example 11)

Brackish water having a high boron concentration was used as the raw liquid. One separation membrane module (1) and one separation membrane module (2) were prepared in the same manner as in Example 2. Using these, pumps and valves were prepared, and piping was connected as shown in Fig. 1. When the valve was adjusted such that the total solution recovery rate was 95% and the recovery rate of the separation membrane 1 was about 95%, and evaluation was performed under the conditions given in Table 2, the results were as shown in Table 2. The circulation ratio of the purified liquid was 0.80. It was possible to selectively separate only boron from the brackish water having a high boron concentration while retaining the electrolyte component.

(Comparative Example 1)

Separation membrane modules the same as those in Example 2 were prepared. As shown in Fig. 1, pumps and valves were prepared, and piping was connected. The permeate liquid circulation valve was adjusted such that all the permeate liquid from the separation membrane module (2) was mixed with the concentrated liquid from the separation membrane module (1) (that is, the permeate liquid from the separation membrane module 2 was not circulated to the raw liquid to the separation membrane module (1)), and formed a purified liquid. When the valve was adjusted such that the total solution recovery rate was 97% and the maximum operation pressure was 5.5 MPa because the maximum operation pressure of the apparatus was 5.5 MPa, and evaluation was performed under the conditions given in Table 3, the results were as shown in Table 3. To increase the urea removal rate of the entire system, it is necessary to increase the recovery rate of the separation membrane module (1) in order to increase the urea permeation amount in the separation membrane module (1). However, when the circulation is not performed in the waste dialysate treatment method of Fig. 1, the osmotic pressure difference of the separation membrane module (1) increases, so that the recovery rate cannot be increased, and a urea removal rate cannot be obtained.

[Table 3]

[Table 3]

| | | | Comparative Example 1 | Comparative Example 2 | Comparative Example 3 |
|---|---|---|---|---|---|
| Separation membrane 1 | | Type | PA membrane A | PA membrane F | PA membrane A |
| | | Electrolyte removal rate [%] | 97.0 | 99.0 | 97.0 |
| | | Urea removal rate [%] | 18.0 | 58.0 | 18.0 |
| | | Module type | I-Type | I-Type | I-Type |
| | | Total membrane area [$m^2$] | 4.0 | 8.0 | 8.0 |

(continued)

|  |  |  | Comparative Example 1 | Comparative Example 2 | Comparative Example 3 |
|---|---|---|---|---|---|
| Separation membrane 2 | | Type | PA membrane D | PA membrane D | PA membrane G |
| | | Electrolyte removal rate [%] | 99.6 | 99.6 | 98.5 |
| | | Urea removal rate [%] | 90.0 | 90.0 | 75.0 |
| | | Module type | I-Type | I-Type | I-Type |
| | | 2 Total membrane area [m$^2$] | 4 | 8 | 8 |
| | | Permeate liquid circulation ratio | 0.00 | 0.90 | 0.90 |
| Solution treatment method | | | Fig. 1 | Fig. 1 | Fig. 1 |
| Raw liquid treatment amount [L/min] | | | 0.51 | 0.51 | 0.51 |
| Solution recovery rate [%] | | | 97.0 | 97.0 | 97.0 |
| Operation results | | Electrolyte recovery rate [%] | 93.2 | 95.2 | 86.8 |
| | | Urea removal rate [%] | 53.2 | 64 . 7 | 62.8 |
| | | Potassium ion removal rate [%] | 7 . 9 | 7.1 | 14.7 |
| | | Separation membrane module 1 Maximum operation pressure [MPa] | 5.50 | 5.50 | 5.50 |
| | | Separation membrane module 2 Maximum operation pressure [MPa] | 2.77 | 3.74 | 4.85 |
| | | Separation membrane module 1 Maximum pump flow rate [L/min] | 0.51 | 4.19 | 4.27 |
| | | Separation membrane module 2 Maximum pump flow rate [L/min] | 0.43 | 4.11 | 4.19 |

(Comparative Example 2)

Separation membrane modules were prepared in the same manner as in Example 1 except that the separation membrane used for the separation membrane module (1) was the PA membrane F. As shown in Fig. 1, pumps and valves were prepared, and piping was connected. When the valve was adjusted such that the total solution recovery rate was 90% and the maximum operation pressure was 5.5 MPa because the maximum operation pressure of the apparatus was 5.5 MPa, and evaluation was performed under the conditions given in Table 3, the results were as shown in Table 3. Since the urea removal rate in the separation membrane module (1) is high, a prescribed urea removal rate cannot be obtained.

(Comparative Example 3)

Separation membrane modules were prepared in the same manner as in Example 1 except that the separation membrane used for the separation membrane module (2) was the PA membrane G. As shown in Fig. 1, pumps and valves were prepared, and piping was connected. When the valve was adjusted such that the water recovery rate of the entire process was 97% and the maximum operation pressure was 5.5 MPa because the maximum operation pressure of the apparatus was 5.5 MPa, and evaluation was performed under the conditions given in Table 3, the results were as shown in Table 3. Since the urea removal rate in the separation membrane module (2) is low, a prescribed urea removal rate cannot be obtained.

INDUSTRIAL APPLICABILITY

The present invention is suitably used for regeneration treatment of waste dialysate, removal of boron from brackish water, and so on.

DESCRIPTION OF REFERENCE SIGNS

1: Separation membrane module
2: Separation membrane module
3: Feed pump of separation membrane module (1)
4: Concentrated liquid valve of separation membrane module (1)
5: Feed pump of separation membrane module (2)
6: Concentrated liquid valve of separation membrane module (2)
7: Permeate liquid flow rate distribution valve of separation membrane module (2)
8: Permeate liquid check valve of separation membrane module (2)
101: Raw liquid (waste dialysate)
102: Purified liquid
103: Discharge liquid
104: Feed liquid to separation membrane module (1)
105: Concentrated liquid from separation membrane module (1)
106: Permeate liquid from separation membrane module (1)
107: Feed liquid to separation membrane module (2)
108: Concentrated liquid from separation membrane module (2)
109: Permeate liquid from separation membrane module (2)
110: Permeate liquid circulation from separation membrane module (2)
201: I-Type separation membrane element
202: Perforated central tube
203: Separation membrane
204: Feed-side channel material
205: Permeate-side channel material
206: Feed liquid
207: Concentrated liquid
208: Permeate liquid
209: U-turn cap
210: Sealing portion
211: Feed-side face of separation membrane
213: I-Type separation membrane body
214: Reverse L-type separation membrane body
215: L-type separation membrane body
216: Separation membrane module
217: Permeate liquid discharge port
218: Feed liquid feed port
219: Concentrated liquid discharge port
220: Pressure vessel
221: Brine seal
222: Reverse L-type separation membrane element
223: L-type separation membrane element
224: U-Turn-type (I-type-reverse L-type) separation membrane element
301: Waste dialysate treatment unit
302: Dialyzer
303: Dialysis membrane
304: Regenerated dialysate

305: Adjustment solution
306: Blood
307: Waste dialysate

**Claims**

1. A solution treatment method comprising treating a raw liquid with a separation membrane 1 and a separation membrane 2 which each separate a feed liquid into a permeate liquid and a concentrated liquid, and separating the raw liquid into a purified liquid containing water and an electrolyte and a discharge liquid containing a low-molecular-weight nonelectrolyte molecule, wherein

   the raw liquid is a solution containing at least an electrolyte in an amount of 1000 mg/L or more and containing a low-molecular-weight nonelectrolyte molecule having a molecular weight of 70 or less,
   a permeate liquid from the separation membrane 1 is fed to the separation membrane 2 and subjected to separation treatment,
   the separation membrane 1 exhibits, when a solution 1 with pH 7 in which 10000 mg/L of sodium chloride (NaCl) and 250 mg/L of urea are mixed is fed thereto at 36°C and a pressure of 1.2 Mpa, an NaCl removal rate of 90% or more, the NaCl removal rate being defined by a reduction rate of a component concentration in a permeated solution relative to a component concentration in a feed liquid,
   the separation membrane 2 exhibits when a solution 2 with pH 7 in which 1000 mg/L of sodium chloride (NaCl) and 700 mg/L of urea are mixed is fed thereto at 36°C and a pressure of 1.8 Mpa, a urea removal rate of 85% or more, the urea removal rate being defined by a reduction rate of a component concentration in a permeated solution relative to a component concentration in a feed liquid,
   a difference between a urea removal rate of the separation membrane 2 under the evaluation conditions of the separation membrane 2 specified above and a urea removal rate of the separation membrane 1 under the evaluation conditions of the separation membrane 1 specified above is 40% point or more, and
   the solution treatment method satisfies any one of the following requirements (i) and (ii):

      (i) a part of a permeate liquid from the separation membrane 2 is mixed with the raw liquid and then fed to the separation membrane 1, and a whole or a part of the remainder of the permeate liquid from the separation membrane 2 is mixed with a concentrated liquid from the separation membrane 1 to afford the purified liquid; and
      (ii) a whole of a permeate liquid from the separation membrane 2 is mixed with the raw liquid and then fed to the separation membrane 1 to afford a concentrated liquid from the separation membrane 1 as the purified liquid.

2. The solution treatment method according to claim 1, wherein the NaCl removal rate of the separation membrane 1 under the evaluation conditions of the separation membrane 1 is 99% or less.

3. The solution treatment method according to claim 1 or 2, wherein the raw liquid is a waste dialysate resulting from artificial dialysis treatment, and the low-molecular-weight nonelectrolyte molecule is urea.

4. The solution treatment method according to claim 1 to 3, wherein in the requirement (i), a ratio of an amount of the permeate liquid from the separation membrane 2 to be mixed with the raw liquid to a whole amount of the permeate liquid from the separation membrane 2 is 0.6 or more.

5. The waste dialysate treatment method according to any one of claims 1 to 4, wherein a ratio of an amount of the purified liquid to an amount of the raw liquid is 75% or more.

6. A solution treatment unit comprising a separation membrane 1 and a separation membrane 2 each of which separates a feed liquid into a permeate liquid and a concentrated liquid, and being configured to separate a raw liquid into a purified liquid containing water and an electrolyte and a discharge liquid containing a low-molecular-weight nonelectrolyte molecule, wherein

   the raw liquid is a solution containing at least an electrolyte in an amount of 1000 mg/L or more and containing a low-molecular-weight nonelectrolyte molecule having a molecular weight of 70 or less,
   a permeate liquid line from the separation membrane 1 is connected to a feed liquid line to the separation membrane 2,

the separation membrane 1 exhibits, when a solution 1 with pH 7 in which 10000 mg/L of sodium chloride (NaCl) and 250 mg/L of urea are mixed is fed thereto at 36°C and a pressure of 1.2 Mpa, an NaCl removal rate of 90% or more, the NaCl removal rate being defined by a reduction rate of a component concentration in a permeated solution relative to a component concentration in a feed liquid,

the separation membrane 2 exhibits, when a solution 2 with pH 7 in which 1000 mg/L of sodium chloride (NaCl) and 700 mg/L of urea are mixed is fed thereto at 36°C and a pressure of 1.8 Mpa, a urea removal rate of 85% or more, the urea removal rate being defined by a reduction rate of a component concentration in a permeated solution relative to a component concentration in a feed liquid,

a difference between a urea removal rate of the separation membrane 2 under the evaluation conditions of the separation membrane 2 specified above and a urea removal rate of the separation membrane 1 under the evaluation conditions of the separation membrane 1 specified above is 40% point or more, and

the solution treatment unit satisfies any one of the following requirements (i) and (ii):

(i) a permeate liquid line from the separation membrane 2 is branched into at least two lines, at least one of the branched lines is connected to a line of the raw liquid, a line of a mixed liquid of the raw liquid and a permeate liquid from the separation membrane 2 is connected to a feed line to the separation membrane 1, and at least one of the remaining branched lines is connected to a concentrated liquid line from the separation membrane 1 to form a purified liquid line; and

(ii) a permeate liquid line from the separation membrane 2 is connected to a line of the raw liquid, a line of a mixed liquid of the raw liquid and a permeate liquid from the separation membrane 2 is connected to a feed line to the separation membrane 1, and a concentrated liquid line from the separation membrane 1 serves as a purified liquid line.

7. The solution treatment unit according to claim 6, wherein the NaCl removal rate of the separation membrane 1 under the evaluation conditions of the separation membrane 1 is 99% or less.

8. The solution treatment unit according to claim 6 or 7, wherein the raw liquid is a waste dialysate resulting from artificial dialysis treatment, and the low-molecular-weight nonelectrolyte molecule is urea.

9. The solution treatment unit according to any one of claims 6 to 8, wherein the separation membranes 1 and 2 are each wound around a perforated central tube to constitute a spiral-type separation membrane element.

10. The solution treatment unit according to claim 9, wherein the separation membrane element has a feed liquid feed part or a concentrated liquid discharge part at an outer peripheral end part in a direction perpendicular to the longitudinal direction of the perforated central tube.

【Fig.1】

【Fig. 2】

【Fig. 3】

【Fig. 4】

213

length W

210    211

205

length L

207

206    204

208

direction perpendicular
to the longitudinal
direction of perforated
central tube

208

210
winding direction

202

longitudinal direction of
perforated central tube

【Fig. 5】

winding direction

【Fig. 6】

winding direction

【Fig. 7】

【Fig. 8】

【Fig. 9】

【Fig. 10】

【Fig. 11】

【Fig. 12】

【Fig. 13】

【Fig. 14】

## INTERNATIONAL SEARCH REPORT

International application No.

**PCT/JP2021/043741**

| A. | CLASSIFICATION OF SUBJECT MATTER |
|---|---|

***B01D 61/02***(2006.01)i; ***B01D 61/26***(2006.01)i; ***B01D 61/32***(2006.01)i; ***B01D 61/58***(2006.01)i; ***A61M 1/16***(2006.01)i
FI:    B01D61/02 500; B01D61/26; B01D61/32; B01D61/58; A61M1/16 190

According to International Patent Classification (IPC) or to both national classification and IPC

| B. | FIELDS SEARCHED |
|---|---|

Minimum documentation searched (classification system followed by classification symbols)

B01D61/02; B01D61/26; B01D61/32; B01D61/58; A61M1/16

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); Japio-GPG/FX

| C. | DOCUMENTS CONSIDERED TO BE RELEVANT |
|---|---|

| Category* | Citation of document, with indication, where appropriate, of the relevant passages | Relevant to claim No. |
|---|---|---|
| A | WO 2020/218571 A1 (TORAY INDUSTRIES, INC.) 29 October 2020 (2020-10-29) <br> claims 1-9, fig. 1-4 | 1-10 |
| A | WO 2009/083011 A2 (MOHAMED, Fahim) 09 July 2009 (2009-07-09) <br> claims 1-17, fig. 1-8 | 1-10 |
| A | US 2017/0065762 A1 (AQUAPOTEN COMPANY LIMITED) 09 March 2017 (2017-03-09) <br> claims 1-21, fig. 1-7 | 1-10 |
| A | JP 2014-205051 A (FRESENIUS MEDICAL CARE HOLDINGS, INC.) 30 October 2014 (2014-10-30) <br> claims 1-4, fig. 1-13 | 1-10 |

☐ Further documents are listed in the continuation of Box C.    ☑ See patent family annex.

| | |
|---|---|
| *    Special categories of cited documents: <br> "A"  document defining the general state of the art which is not considered to be of particular relevance <br> "E"  earlier application or patent but published on or after the international filing date <br> "L"  document which may throw doubts on priority claim(s) or which is cited to establish the publication date of another citation or other special reason (as specified) <br> "O"  document referring to an oral disclosure, use, exhibition or other means <br> "P"  document published prior to the international filing date but later than the priority date claimed | "T"  later document published after the international filing date or priority date and not in conflict with the application but cited to understand the principle or theory underlying the invention <br> "X"  document of particular relevance; the claimed invention cannot be considered novel or cannot be considered to involve an inventive step when the document is taken alone <br> "Y"  document of particular relevance; the claimed invention cannot be considered to involve an inventive step when the document is combined with one or more other such documents, such combination being obvious to a person skilled in the art <br> "&"  document member of the same patent family |

| Date of the actual completion of the international search | Date of mailing of the international search report |
|---|---|
| **12 January 2022** | **25 January 2022** |

| Name and mailing address of the ISA/JP | Authorized officer |
|---|---|
| **Japan Patent Office (ISA/JP)** <br> **3-4-3 Kasumigaseki, Chiyoda-ku, Tokyo 100-8915** <br> **Japan** | |
| | Telephone No. |

Form PCT/ISA/210 (second sheet) (January 2015)

**INTERNATIONAL SEARCH REPORT**
**Information on patent family members**

International application No.

**PCT/JP2021/043741**

| Patent document cited in search report | | | Publication date (day/month/year) | Patent family member(s) | | | Publication date (day/month/year) |
|---|---|---|---|---|---|---|---|
| WO | 2020/218571 | A1 | 29 October 2020 | (Family: none) | | | |
| WO | 2009/083011 | A2 | 09 July 2009 | (Family: none) | | | |
| US | 2017/0065762 | A1 | 09 March 2017 | WO | 2015/124716 | A1 | |
| | | | | claims 1-25, fig. 1-7 | | | |
| | | | | CN | 106573094 | A | |
| | | | | EP | 3110532 | A1 | |
| JP | 2014-205051 | A | 30 October 2014 | US | 2011/0060273 | A1 | |
| | | | | claims 1-24, fig. 1-13 | | | |
| | | | | WO | 2011/031610 | A1 | |
| | | | | EP | 2475407 | A1 | |
| | | | | CA | 2772629 | A1 | |
| | | | | CN | 102481400 | A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H09290275 A
- JP 2003088863 A
- JP 2014204958 A
- JP 2014530643 A
- WO 2020218571 A

**Non-patent literature cited in the description**

- Experimental Chemistry Lecture. Maruzen Co., Ltd, 1992, vol. 14, 485
- **NAKANISHI.** Journal of Polymer Science, Part B: Polymer Physics. John Wiley & Sons, Inc, 1989, vol. 27, 1419
- Radiation Physics and Chemistry. Pergamon, 2000, vol. 58, 603